(19) **Europäisches Patentamt / European Patent Office / Office européen des brevets**

(11) **EP 4 663 388 A1**

(12) **EUROPEAN PATENT APPLICATION**
published in accordance with Art. 153(4) EPC

(43) Date of publication:
17.12.2025   Bulletin 2025/51

(21) Application number: 24753078.5

(22) Date of filing: 18.01.2024

(51) International Patent Classification (IPC):
$B29C\ 64/314^{(2017.01)}$   $A61C\ 13/15^{(2006.01)}$
$A61K\ 6/802^{(2020.01)}$   $A61K\ 6/831^{(2020.01)}$
$B29C\ 64/165^{(2017.01)}$   $B29C\ 64/35^{(2017.01)}$
$B29C\ 64/379^{(2017.01)}$   $B33Y\ 10/00^{(2015.01)}$
$B33Y\ 40/20^{(2020.01)}$   $B33Y\ 70/10^{(2020.01)}$
$C08K\ 3/013^{(2018.01)}$   $C08L\ 101/12^{(2006.01)}$

(52) Cooperative Patent Classification (CPC):
A61C 19/003; A61K 6/802; A61K 6/831;
B29C 64/165; B29C 64/314; B29C 64/35;
B29C 64/379; B33Y 10/00; B33Y 40/20;
B33Y 70/10; C08K 3/013; C08L 101/12

(86) International application number:
PCT/JP2024/001193

(87) International publication number:
WO 2024/166632 (15.08.2024 Gazette 2024/33)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 09.02.2023 JP 2023018503

(71) Applicant: Tokuyama Dental Corporation
Tokyo 110-0016 (JP)

(72) Inventors:
• SAKATA, Eibu
  Tokyo 110-0016 (JP)
• NAKASHIMA, Kei
  Tokyo 110-0016 (JP)

(74) Representative: HGF
HGF Europe LLP
Neumarkter Straße 18
81673 München (DE)

(54) **CURABLE COMPOSITION FOR THREE-DIMENSIONAL STEREOLITHOGRAPHY, METHOD FOR PRODUCING SAME, METHOD FOR PRODUCING THREE-DIMENSIONAL STEREOLITHOGRAPHIC MODEL, AND METHOD FOR PRODUCING DENTAL RESTORATION**

(57) The present invention provides a curable composition for three-dimensional photofabrication, the curable composition being used in a vat photopolymerization method and containing 100 parts by mass of a polymerizable monomer component (A), 40 parts by mass to 400 parts by mass of an inorganic filler (B) that is composed of a single kind or plural kinds of inorganic particulate matter, and 0.01 part by mass to 5 parts by mass of a photopolymerization initiator (C), wherein: 80% or more of all primary particles that constitute the inorganic filler (B) have a particle diameter of 0.05 μm to 5.0 μm; and a sample, which is formed of this curable composition for three-dimensional photofabrication and has a thickness of 0.5 mm, has a transmittance of 1.00% to 50.00% with respect to activation light. The present invention also provides a method for producing this curable composition for three-dimensional photofabrication. The present invention also provides a method for producing a three-dimensional photofabricated product and a method for producing a dental restoration, each using this curable composition for three-dimensional photofabrication.

# FIG .1

**Description**

TECHNICAL FIELD

The present disclosure relates to a curable composition for three-dimensional photofabrication, a method for producing the same, a method for producing a three-dimensional photofabricated product, and a method for producing a dental restoration.

BACKGROUND ART

A technique for shaping a three-dimensional photofabricated product by irradiating a photocurable composition (also referred to as photocurable resin or photocurable resin composition) containing a polymerizable monomer and a photopolymerization initiator with light (activating light) for activating the photopolymerization initiator and curing the photocurable composition is known as a photofabrication method. There are several methods as the photofabrication method, and among these methods, a vat photopolymerization process is widely used because the device is relatively inexpensive and a fabricated product having a smooth surface can be produced with high accuracy.

In the vat photopolymerization process, a three-dimensional object as a production target is generally obtained as follows. First, a height direction of the three-dimensional object is digitized and ordinated based on three-dimensional shape data indicating a shape of the three-dimensional object, and two-dimensional shape data indicating a cross-sectional shape of the three-dimensional object at each ordinated height is generated. Next, a liquid photocurable composition held in a vat is irradiated with activating light at a predetermined position determined in advance based on the two-dimensional shape data, so that the liquid photocurable composition present at the position is selectively primarily cured to form a fabricated layer having the cross-sectional shape, and the fabricated layer having the cross-sectional shape at each height according to the order of ordinating is sequentially formed and stacked to obtain a stack having a shape corresponding to the shape of the three-dimensional object. Next, the stack is washed with an organic solvent as necessary, and then secondarily cured to obtain a target product.

In the dental field, dental restorations such as dentures and crown prostheses need to be produced highly precisely such that the dental restorations have a unique shape in accordance with the situation in the oral cavity of each individual patient. For this reason, it has been studied to produce a dental restoration by photofabrication according to a vat photopolymerization process based on computer aided design (CAD) data designed by using digital data obtained by oral scanning or the like.

The dental prostheses used in the oral cavity is required not only to have high dimensional (shape) accuracy as described above, but also to have high mechanical strength that may sufficiently withstand a load applied during chewing. In this regard, when an inorganic filler is blended in the photocurable composition, polymerization shrinkage, which is one of the causes of a decrease in accuracy, can be reduced, and the mechanical strength and the surface hardness of the cured product can be improved. Therefore, it is considered that a vat photopolymerization process by using a photocurable composition blended with an inorganic filler is suitable for photofabrication of dental prostheses, and a curable composition for three-dimensional photofabrication containing such an inorganic filler is also proposed.

For example, Patent Document 1 discloses a composition containing a polymerizable monomer (a), an ultraviolet light absorbing inorganic particle (b), and a photopolymerization initiator (c), as a composition for optical fabrication, which is excellent in shaping accuracy, mechanical characteristics, and transparency, and is particularly suitable for a dental material. Patent Document 2 discloses, a composition containing a transparent resin and two or more types of transparent particles having different refractive indexes and Abbe numbers, as a resin composition capable of producing a cured product (three-dimensional fabricated product) having excellent designability. Patent Document 3 discloses, as a resin composition for photofabrication capable of obtaining a three-dimensional fabricated product having high strength, high elasticity, and excellent wear resistance, a composition containing a urethanated (meth) acrylic compound (a), a (meth) acrylic amide compound (b), a photopolymerization initiator (c), and spherical inorganic particles (d) having an average particle diameter of 0.75 $\mu$m to 10 $\mu$m, in which a content of the spherical inorganic particles (d) is 50 parts by mass to 400 parts by mass with respect to 100 parts by mass of the total amount of the polymerizable monomers.

Citation List

Patent Document

Patent Document 1: PCT International Publication No. WO2018/074380
Patent Document 2: Japanese Unexamined Patent Application, Publication No. 2020-180171

Patent Document 3: Japanese Unexamined Patent Application, Publication No. 2022-41276
Patent Document 4: Japanese Patent No. 3917204

DISCLOSURE OF THE INVENTION

Problems to be Solved by the Invention

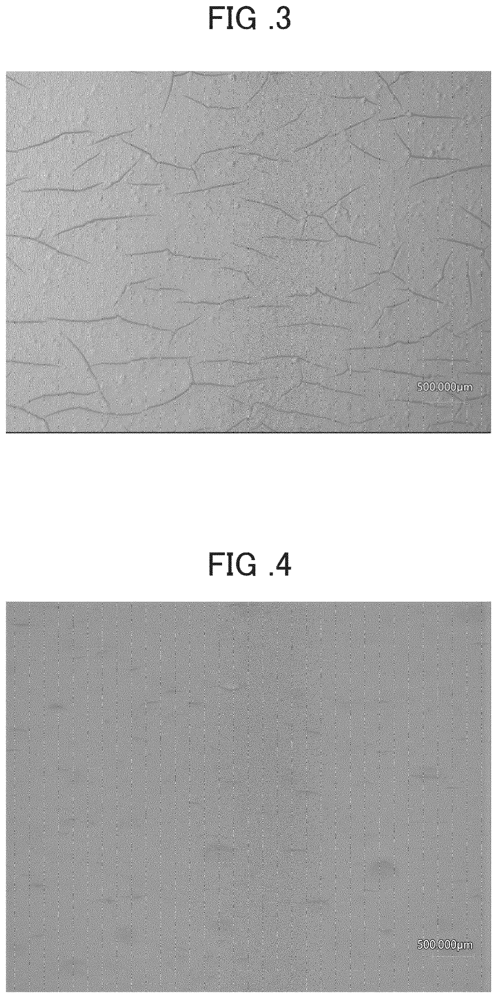

In Patent Documents 1 to 3, since a composition in which an inorganic filler is added to a polymerizable monomer is used, it is possible to obtain a fabricated product having good mechanical strength, elastic modulus, and wear resistance. However, since the composition for optical fabrication described in Patent Document 1 has low fluidity, the shape of an applicable fabricated product may be limited. In addition, in the resin composition described in Patent Document 2, when the resin composition is left to stand for a long period of time, light-transmitting particles (glass filler or the like) contained in the composition may precipitate. Further, in the resin composition for photofabrication described in Patent Document 3, it has been revealed that fine cracks which are difficult to visually distinguish are often generated on a surface of the fabricated product (see FIG. 4). When such a fabricated product having fine cracks is used as a dental prosthesis, there is a possibility that the dental prosthesis may become a starting point of breaking in the oral cavity, which is a problem.

Therefore, an object of the present disclosure is to provide a technique capable of producing a three-dimensional fabricated product with high accuracy and high strength without generating cracks on a surface at the time of producing the three-dimensional fabricated product by photofabrication by a vat photopolymerization process using a curable composition for three-dimensional photofabrication in which a certain amount of an inorganic filler is blended for high strength.

Means for Solving the Problems

A first aspect of the present disclosure is a curable composition for three-dimensional photofabrication to be used as a liquid photocurable composition in a vat photopolymerization process for producing a three-dimensional photofabricated product by irradiating a predetermined position of the liquid photocurable composition held in a vat with activating light (hereinafter also referred to as "specific activating light") including light having a specific wavelength $\lambda$ (nm) in an ultraviolet light range or a visible light range and selectively curing the liquid photocurable composition present at the position, the curable composition containing:

100 parts by mass of a polymerizable monomer component (A); 40 parts by mass to 400 parts by mass of an inorganic filler (B) containing a single type or a plurality of types of inorganic particulate matters; and 0.01 parts by mass to 5 parts by mass of a photopolymerization initiator (C) having a function of initiating photopolymerization by irradiation with the specific activating light,
80% or more of all primary particles constituting the inorganic filler (B) being particles having a particle diameter of 0.05 $\mu$m to 5.0 $\mu$m in a particle size distribution of the inorganic filler (B) measured by microscopy method using a scanning microscope,
a transmittance to the specific activating light measured for a sample having a thickness of 0.5 mm and containing the curable composition for three-dimensional photofabrication being 1.00 to 50.00 (%).

It is preferable that the curable composition for three-dimensional photofabrication according to the present disclosure contains 0.01 parts by mass to 2.5 parts by mass of an activating light absorbent (D) having a function of absorbing the specific activating light and having no photopolymerization initiation ability, and

the polymerizable monomer component (A) and the inorganic filler (B) have a value of a light scattering index Sc (%) of 10 (%) or less,
the light scattering index Sc (%) being determined by the following formula based on an intensity of transmitted light in a specific emission angle direction obtained by measurement using a variable angle spectrophotometer in which a sample having a thickness of 0.5 mm and containing a base composition is vertically irradiated with measurement light including light having the specific wavelength $\lambda$ (nm), including light having a wavelength in a range of $\lambda \pm 50$ (nm) as a main component, and having a maximum intensity in the range, the base composition being a composition consisting of the polymerizable monomer component (A) and the inorganic filler (B) and having a composition ratio of the components same as a composition ratio of the curable composition for three-dimensional photofabrication according to the present disclosure,

$$Sc = \{(I_{70} + I_{75} + I_{80})/(I_0 \times 3)\} \times 100$$

wherein $I_0$, $I_{70}$, $I_{75}$, and $I_{80}$ indicate intensities of the transmitted light in directions of emission angles of 0°, 70°, 75°, and 80°, respectively (hereinafter, this aspect is also referred to as a "specific blending aspect").

In the curable composition for three-dimensional photofabrication according to the present disclosure, when a particle diameter of each particle constituting the inorganic filler (B) is defined as x (nm) and a circumference ratio is defined as $\pi$, it is preferable that a total number of particles having a particle diameter x (nm) within a range of $0.7\,\lambda/\pi$ to $4\,\lambda/\pi$ (nm) is 40% or more of the number of all particles constituting the inorganic filler (B) in a particle size distribution of the inorganic filler (B) measured by microscopy method using a scanning microscope.

In the specific blending aspect, in the inorganic particulate matters constituting the inorganic filler (B), when an inorganic particulate matter consisting of an aggregate of a single type of inorganic particles having a refractive index with respect to a line D at 25°C in a range of 1.500 to 1.550 is defined as a specific inorganic particulate matter (b1) and an inorganic particulate matter consisting of an aggregate of a single type of inorganic particles having a refractive index out of the range is defined as a non-specific inorganic particulate matter (b2), the inorganic filler (B)

(1) contains a single type of the specific inorganic particulate matter (b1),
(2) contains a plurality of types of the specific inorganic particulate matters (b1), at least one of the plurality of types of specific inorganic particulate matters (b1) occupying 10 mass% or more of a total mass of the inorganic filler (B), or
(3) contains 90 mass% or more and less than 100 mass% of a single type or a plurality of types of the specific inorganic particulate matters (b1) and more than 0 mass% and less than 10 mass% of a single type or a plurality of types of the non-specific inorganic particulate matters (b2), at least one of the single type or the plurality of types of specific inorganic particulate matters (b1) occupying 10 mass% or more of a total mass of the inorganic filler (B), a refractive index $n_M$ of the polymerizable monomer component (A) with respect to the line D at 25°C is 1.490 to 1.550, and when a refractive index at which a difference with $n_m$ is largest among the refractive indexes of at least one of the specific inorganic particulate matters (b1) occupying 10 mass% or more of the inorganic filler (B) is defined as $n_F$, an absolute value of a difference between $n_F$ and $n_M$ $|n_F - n_M|$ is 0.035 or less.

A second aspect of the present disclosure is a method for producing a curable composition for three-dimensional photofabrication, the method including:

a mixing step of mixing 100 parts by mass of a polymerizable monomer component (A), 40 parts by mass to 400 parts by mass of an inorganic filler (B) containing a single type or a plurality of types of inorganic particulate matters, 0.01 parts by mass to 5 parts by mass of a photopolymerization initiator (C) having a function of initiating photopolymerization by irradiation with specific activating light, and 0.01 parts by mass to 2.5 parts by mass of an activating light absorbent (D) having a function of absorbing the specific activating light and having no photopolymerization initiation ability,
in the mixing step, the polymerizable monomer component (A) and the inorganic filler (B) satisfy the following conditions 1 to 4:

condition 1: when a particle diameter of each particle constituting the inorganic filler (B) is defined as x (nm) and a circumference ratio is defined as $\pi$, a total number of particles having a particle diameter x (nm) within a range of $0.7\,\lambda/\pi$ to $4\,\lambda/\pi$ (nm) is 40% or more of the number of all particles constituting the inorganic filler (B) in a particle size distribution of the inorganic filler (B) measured by microscopy method using a scanning microscope;
condition 2: in the inorganic particulate matters constituting the inorganic filler (B), when an inorganic particulate matter consisting of an aggregate of a single type of inorganic particles having a refractive index with respect to a line D at 25°C in a range of 1.500 to 1.550 is defined as a specific inorganic particulate matter (b1) and an inorganic particulate matter consisting of an aggregate of a single type of inorganic particles having a refractive index out of the range is defined as a non-specific inorganic particulate matter (b2), the inorganic filler (B)

(1) contains a single type of the specific inorganic particulate matter (b1),
(2) contains a plurality of types of the specific inorganic particulate matters (b1), at least one of the plurality of types of specific inorganic particulate matters (b1) occupying 10 mass% or more of a total mass of the inorganic filler (B), or
(3) contains 90 mass% or more and less than 100 mass% of a single type or a plurality of types of the specific inorganic particulate matters (b1) and more than 0 mass% and less than 10 mass% of a single type or a plurality of types of the non-specific inorganic particulate matters (b2), at least one of the single type or the plurality of types of specific inorganic particulate matters (b1) occupying 10 mass% or more of a total mass of the inorganic filler (B);

condition 3: a refractive index $n_M$ of the polymerizable monomer component (A) with respect to the line D at 25°C is 1.490 to 1.550; and

condition 4: when a refractive index at which a difference with $n_M$ is largest among the refractive indexes of at least one of the specific inorganic particulate matters (b1) occupying 10 mass% or more of the inorganic filler (B) is defined as $n_F$, an absolute value of a difference between $n_F$ and $n_M$ $|n_F - n_M|$ is 0.035 or less.

In the method for producing the curable composition for three-dimensional photofabrication according to the present disclosure, it is preferable that the polymerizable monomer component (A) and the inorganic filler (B) satisfying the conditions 1 to 4 are used to separately prepare a base composition containing a composition which consists of the polymerizable monomer component (A) and the inorganic filler (B) and in which a composition ratio of the components is same as a composition ratio of the curable composition for three-dimensional photofabrication as a production target, and the polymerizable monomer component (A) and the inorganic filler (B) found to have a value of a light scattering index Sc (%) of 10% or more are used in the mixing step, the light scattering index being determined by the following formula based on an intensity of transmitted light in a specific emission angle direction obtained by measurement using a variable angle spectrophotometer in which a sample having a thickness of 0.5 mm and containing the base composition is vertically irradiated with measurement light including light having the specific wavelength $\lambda$ (nm), including light having a wavelength in a range of $\lambda \pm 50$ (nm) as a main component, and having a maximum intensity in the range:

$$Sc = \{(I_{70} + I_{75} + I_{80}) / (I_0 \times 3)\} \times 100$$

wherein $I_0$, $I_{70}$, $I_{75}$, and $I_{80}$ indicate intensities of the transmitted light in directions of emission angles of 0°, 70°, 75°, and 80°, respectively.

A third aspect of the present disclosure is a method for producing a three-dimensional photofabricated product, which is a method for producing a three-dimensional photofabricated product by irradiating a predetermined position of a liquid photocurable composition held in a vat with specific activating light and selectively curing the liquid photocurable composition present at the position, the method including:

a shaping step of digitizing and ordinating a height direction of a three-dimensional object based on three-dimensional shape data indicating a shape of the three-dimensional object,

generating two-dimensional shape data indicating a cross-sectional shape of the three-dimensional object at each ordinated height, irradiating the liquid photocurable composition held in the vat with the specific activating light at a predetermined position determined in advance based on the two-dimensional shape data, thereby selectively primarily curing the liquid photocurable composition present at the position, forming a fabricated layer having the cross-sectional shape, and sequentially forming and stacking the fabricated layer having the cross-sectional shape at each height according to an order of the ordinating, to obtain a stack having a shape corresponding to the shape of the three-dimensional object;

a washing step of washing, with an organic solvent, the stack obtained in the shaping step; and

a secondary curing step of secondarily curing the stack washed in the washing step by performing additional activating light irradiation or a heating treatment, or both of the additional activating light irradiation and the heating treatment, the curable composition for three-dimensional photofabrication according to the present disclosure being used as the liquid photocurable composition.

A fourth aspect of the present disclosure is a method for producing a dental restoration, including: producing the dental restoration by the method for producing a three-dimensional photofabricated product according to the present disclosure.

Effects of the Invention

With the curable composition for three-dimensional photofabrication according to the present disclosure, it is possible to produce a three-dimensional photofabricated product having excellent mechanical strength and good fabrication accuracy while preventing a decrease in fluidity and precipitation of an inorganic filler and preventing generation of cracks on a surface of a cured product.

BRIEF DESCRIPTION OF THE DRAWINGS

FIG. 1 is a diagram illustrating a state where a surface of a three-dimensional photofabricated product obtained in Example 7 is observed with an optical microscope (magnification of 50 times).

FIG. 2 is a diagram illustrating a state where a surface of a three-dimensional photofabricated product obtained in Example 11 is observed with an optical microscope (magnification of 50 times).

FIG. 3 is a diagram illustrating a state where a surface of a three-dimensional photofabricated product obtained in Comparative Example 1 is observed with an optical microscope (magnification of 50 times).

FIG. 4 is a diagram illustrating a state where a surface of a three-dimensional photofabricated product obtained in Comparative Example 9 is observed with an optical microscope (magnification of 50 times).

FIG. 5 is a graph illustrating a wavelength distribution (relative spectral distribution) of measurement light used for measuring a light scattering index Sc in examples and comparative examples.

PREFERRED MODE FOR CARRYING OUT THE INVENTION

As described above, in a photocurable composition in which a certain amount of an inorganic filler is blended in order to improve mechanical strength and surface hardness of a cured product, there may be a problem of a decrease in fluidity or particle precipitation during storage. In addition, when a fabricated product is produced by a vat photopolymerization process using the resin composition for photofabrication described in Patent Document 3 (although not recognized in Patent Document 3), fine cracks which are difficult to visually determine are often generated on the surface of the fabricated product.

The present inventors have found that these problems may be solved by controlling a particle diameter of particles constituting the inorganic filler to be blended and setting a transmittance to activating light in the composition before curing to a specific range, thereby completing the invention of the present disclosure.

Hereinafter, a curable composition for three-dimensional photofabrication (hereinafter, also simply referred to as "curable composition for photofabrication"), a method for producing the same, a method for producing a three-dimensional photofabricated product, and a method for producing a dental restoration according to the present disclosure will be described. In the present specification, unless otherwise specified, a notation "x to y" using numerical values x and y means "x or more and y or less". In such a notation, when a unit is added to only the numerical value y, the unit is also applied to the numerical value x. In the present specification, the term "(meth) acrylic" means both "acrylic" and "methacrylic". Similarly, the term "(meth)acrylate" means both "acrylate" and "methacrylate", and the term "(meth)acryloyl" means both "acryloyl" and "methacryloyl".

1. Curable Composition for Photofabrication

The curable composition for photofabrication according to the present disclosure is a composition to be used as a liquid photocurable composition at the time of producing a three-dimensional photofabricated product using a vat photopolymerization process, that is, a curable composition for three-dimensional photofabrication by a vat photopolymerization process.

Here, the vat photopolymerization process means a method including a step (hereinafter also referred to as "shaping step") of digitizing and ordinating a height direction of a three-dimensional object from three-dimensional shape data indicating a shape of the three-dimensional object, generating two-dimensional shape data indicating a cross-sectional shape of the three-dimensional object at each ordinated height, irradiating a liquid photocurable composition held in a vat with activating light at a predetermined position determined in advance based on the two-dimensional shape data, so that the liquid photocurable composition present at the position is selectively (primarily) cured to form a fabricated layer having the cross-sectional shape, and sequentially forming and stacking the fabricated layer having the cross-sectional shape at each height according to the order of ordinating to obtain a stack having a shape corresponding to the shape of the three-dimensional object, and as necessary, performing a washing treatment using an organic solvent (hereinafter, step of performing such treatment is also referred to as "washing step") or a secondary curing treatment (hereinafter, step of performing such treatment is also referred to as "secondary curing step") to obtain a three-dimensional photofabricated product having a shape corresponding to the shape of the three-dimensional object.

The curable composition for photofabrication according to the present disclosure contains 100 parts by mass of a polymerizable monomer component (A), 40 parts by mass to 400 parts by mass of an inorganic filler (B) containing a single type or a plurality of types of inorganic particulate matters, and 0.01 parts by mass to 5 parts by mass of a photopolymerization initiator (C) having a function of initiating photopolymerization by irradiation with specific activating light, and in a particle size distribution of the inorganic filler (B) measured by microscopy method using a scanning microscope, 80% or more of all particles constituting the inorganic filler (B) are particles having a particle diameter of 0.05 $\mu$m to 5.0 $\mu$m, and a transmittance to the specific activating light measured for a sample having a thickness of 0.5 mm and containing the curable composition for photofabrication is 1.00 to 50.00 (%).

When the polymerizable monomer component (A) and the inorganic filler (B) are contained at the above-described mass ratio, the strength and the surface hardness of the cured product to be a three-dimensional photofabricated product as a production target may be increased. In addition, when the particles constituting the inorganic filler (B) satisfy the above conditions of the particle diameter, it is possible to prevent an increase in viscosity of the composition and a risk of precipitation of particles during storage.

Here, the fact that 80% or more of all primary particles constituting the inorganic filler (B) are particles having a particle diameter of 0.05 $\mu$m to 5.0 $\mu$m can be found by the particle size distribution of the inorganic filler (B) measured by microscopy method using a scanning microscope. That is, the particle size distribution can be determined by photographing, by a scanning electron microscope, the inorganic filler (B) used for preparing the curable composition for photofabrication according to the present disclosure, measuring the number n of all primary particles (50 or more) observed in a unit field of view of the photograph, and measuring a primary particle diameter (maximum diameter) $X_i$ (nm) of each particle for all primary particles. Here, i in $X_i$ is a natural number of 1 to n, and represents the number of each measured primary particle. The average (primary) particle diameter X (nm) can be calculated by the equation $X = \Sigma X_i/n$ using the total sum $\Sigma X_i$ of the measured particle diameters (of primary particles where i = 1 to n) of all the primary particles.

The inorganic filler (B) may be contained as aggregated particles in which primary particles are aggregated. The particle size distribution of the aggregated particles is not particularly limited as long as the particle size distribution of the primary particles satisfies the above conditions, but in order to prevent the precipitation of the inorganic filler (B), the amount of the aggregated particles of the inorganic filler (B) contained in the curable composition for photofabrication according to the present disclosure is preferably small. An average particle diameter of the inorganic filler (B) containing the aggregated particles of the primary particles measured by a laser diffraction/scattering method is usually 0.05 $\mu$m to 100 $\mu$m, preferably 0.05 $\mu$m to 50 $\mu$m, and more preferably 0.05 $\mu$m to 30 $\mu$m.

In addition, in the curable composition for photofabrication according to the present disclosure, the transmittance to the specific activating light measured for the sample having a thickness of 0.5 mm and containing the curable composition for photofabrication is 1.00 to 50.00 (%), so that it is possible to reduce the risk of generation of cracks on the surface of the cured product.

In general, the reason why cracks are generated on the surface of the cured product is unclear, but the present inventors have found that cracks are generated when the uncured curable composition adhered to the surface of the stack is washed with an organic solvent after the stack is obtained using a photofabrication device. In addition, since the inorganic filler contained in the curable composition for photofabrication contains a large amount of particles having a particle diameter that causes scattering (specifically, Mie scattering or Reyleigh scattering) when irradiated with the activating light, it is considered that the generation of weak activating light (side scattered light) scattered from an optical axis at an irradiation spot causes a very shallow curing depth, and formation of a region (hereinafter also referred to as "interlayer low-crosslinking-density region") where the crosslinking density is relatively small between the layers of the stack also contributes to generation of cracks. That is, permeation of the organic solvent into the interlayer low-crosslinking-density region during washing causes swelling of the region, so that an intermolecular space of a high molecular chain constituting a shaped product is widened, and the strength is temporarily reduced. At this time, it is considered that an internal stress remaining in the shaped product in the shaping step destroys the portion having the reduced strength, thereby causing generation of a crack in the region.

Although the reason why the crack on the surface of the cured product is not recognized in Patent Document 3 is unclear, the present inventors conducted an additional experiment on the resin composition for photofabrication described in Patent Document 3, and found that the resin composition for photofabrication for which cracks are observed on the surface of the cured product has low transmittance to activating light. From this, it is considered that in Patent Document 3, the generation of cracks is overlooked.

In the curable composition for photofabrication according to the present disclosure, the transmittance to the specific activating light is set within the above range, so that the influence of scattering is reduced, and the risk of generation of cracks on the surface of the cured product is reduced. In order to more reliably prevent the generation of cracks and to produce a three-dimensional photofabricated product with high accuracy, the curable composition for photofabrication according to the present disclosure is preferably in the above-described specific blending aspect. In the specific blending aspect, a polymerizable monomer component, an inorganic filler having a particle diameter in which an increase in the viscosity and a risk of precipitation are prevented, a photopolymerization initiator, and an activating light absorbent are contained at a predetermined mass ratio, respectively, and a combination such that the light scattering index Sc falls within a specific range is adopted as a combination of the polymerizable monomer component and the inorganic filler. In the specific blending aspect, light scattering due to the inorganic filler in a direction deviated from the optical axis is prevented and the transmittance to the specific activating light (in state before curing) is set within a specific range, so that generation of cracks can be prevented, and in some cases, the fabrication accuracy can be further improved.

That is, the specific blending aspect is a uniform composition containing 100 parts by mass of a polymerizable monomer component (A), 40 parts by mass to 400 parts by mass of an inorganic filler (B) containing a single type or a plurality of types of inorganic particulate matters, 0.01 parts by mass to 5 parts by mass of a photopolymerization initiator (C) having a function of initiating photopolymerization by irradiation with specific activating light, and 0.01 parts by mass to 2.5 parts by mass of an activating light absorbent (D) having a function of absorbing specific activating light and having no photopolymerization initiation ability, and the following conditions [I] and [II] are satisfied, so that the effects of high strength, crack generation prevention, and high accuracy can be exerted.

[I] The transmittance to the specific activating light measured for the sample having a thickness of 0.5 mm and containing the curable composition for photofabrication is 1.00 to 50.00 (%).

[II] The value of the light scattering index Sc (%) is 10% or less, the light scattering index being determined by the formula $Sc = \{(I_{70} + I_{75} + I_{80})/(I_0 \times 3)\} \times 100$ based on an intensity of transmitted light in a specific emission angle direction obtained by measurement using a variable angle spectrophotometer in which the sample having a thickness of 0.5 mm and containing a base composition is vertically irradiated with measurement light including light having the specific wavelength $\lambda$ (nm), including light having a wavelength in a range of $\lambda \pm 50$ (nm) as a main component, and having a maximum intensity in the range, the base composition being a composition consisting of the polymerizable monomer component (A) and the inorganic filler (B) and having a composition ratio of the components same as a composition ratio of the curable composition for photofabrication. $I_0$, $I_{70}$, $I_{75}$, and $I_{80}$ indicate intensities of the transmitted light in directions of emission angles of 0°, 70°, 75°, and 80°, respectively.

When the curable composition for photofabrication as described in the specific blending aspect does not satisfy the above-described conditions related to the blending components and the blending ratio thereof and does not satisfy the condition of the transmittance to the specific activating light, it is difficult to produce a three-dimensional fabricated product having high strength with high accuracy. When the condition [I] is not satisfied, for example, when the transmittance is less than 1%, since a sufficient curing depth is not obtained, a large number of interlayer low-crosslinking-density regions are formed, and the generation of cracks becomes remarkable. On the other hand, in the composition containing the inorganic filler (B) and the activating light absorbent (D), it is extremely difficult to obtain a system in which the transmittance exceeds 50% while maintaining high fabrication accuracy.

These conditions define conditions to be satisfied by the polymerizable monomer component (A), the inorganic filler (B), the photopolymerization initiator (C), and the activating light absorbent (D), which are components in the specific blending aspect. The transmittance defined by the condition [I] corresponds to the "transmittance to specific activating light in the curable composition for photofabrication in the specific blending aspect in a state before photocuring", and is an index of the curing depth when the curable composition for photofabrication according to the present disclosure is irradiated with the specific activating light. Sc defined by the condition [II] is an index of "the scattering state of light when the curable composition for photofabrication according to the present disclosure is irradiated with the specific activating light", specifically, the state of the side scattered light, and further, the spread of the interlayer low-crosslinking-density region formed thereby. The curing depth and the state of the side scattered light are important for the development of the effect of the curable composition for photofabrication according to the present disclosure, and are determined by the combination of the components to be used. However, since the transmittance defined by the condition [I] and the physical properties of each component affecting Sc defined by the condition [II] are various, it is substantially impossible to directly define a combination of specific substances satisfying these conditions. Therefore, in the specific blending aspect, the components (A), (B), (C), and (D) are defined so as to simultaneously satisfy the conditions [I] and [II], and in the curable composition for photofabrication which does not contain the component (D), the components (A), (B), and (C) are defined so as to simultaneously satisfy the conditions [I] and [II]. The curable composition for photofabrication according to the present disclosure includes a curable composition obtained by the method for producing a curable composition for photofabrication according to the present disclosure described below.

In this way, since it is extremely important to satisfy the conditions [I] and [II] from the viewpoint of crack prevention, first, the description thereof will be made, and then blending components of the curable composition for photofabrication according to the present disclosure, the blending ratio thereof, and the like will be described.

1-1. Regarding Condition [I]

Since the curable composition for photofabrication according to the present disclosure contains a photopolymerization initiator regardless of whether the composition is in the specific blending aspect, there is a concern about the progress of curing by irradiation with light during measurement. In this regard, it is possible to measure the transmittance to the specific activating light before the progress of curing by using a transmission measurement method using a color difference meter described later.

The transmittance of the curable composition for photofabrication according to the present disclosure to the specific activating light can be measured as follows. First, a measurement sample having a thickness of 0.5 mm is prepared by a method in which the curable composition for photofabrication according to the present disclosure is filled in a resin mold (25 mm × 25 mm × 0.5 mm in thickness), then upper and lower surfaces are pressed with a slide glass, and the slide glass is removed after the thickness becomes 0.5 mm. Next, the measurement sample is set in a color difference meter (for example, spectrophotometer SE7700 manufactured by Nippon Denshoku Industries Co., Ltd.), and the transmittance to activating light (for example, light having wavelength of 405 nm or light having wavelength of 385 nm) is measured by transmission measurement using a halogen lamp (measurement wavelength of 380 nm to 780 nm).

As described above, when the transmittance measured in this way is lower than 1.00%, which is a lower limit value, a sufficient curing depth is not obtained, photofabrication is difficult, and cracks are likely to be generated on the fabricated product even when photofabrication is possible. In addition, in a system containing 40 parts by mass or more of an inorganic filler with respect to 100 parts by mass of a polymerizable monomer component and containing a photopolymerization initiator in an amount capable of being cured by photofabrication, it is difficult to obtain a transmittance greatly exceeding 50.00%, which is an upper limit value. In addition, even in a range of transmittance that may be implemented, since the specific activating light is excessively irradiated, a curing reaction excessively occurs, and a shaped product having a shape completely different from the CAD data is provided. From the viewpoint of crack generation prevention and the shape of the shaped product, the transmittance is preferably 2.00% to 30.00%, and more preferably 5.00% to 20.00%.

The transmittance tends to increase as the light transmittance of the polymerizable monomer component and the inorganic filler increases and as the difference between the refractive index of the polymerizable monomer component and the refractive index of the inorganic filler decreases, and is affected by a blending ratio of the polymerizable monomer component and the inorganic filler when the light transmittance of the polymerizable monomer component and the light transmittance of the inorganic filler are different. In general, since the transmittance of the polymerizable monomer component is considerably higher than 5.00%, when a blending amount of the inorganic filler is within a defined range, it is possible to sufficiently set the transmittance not only to 1.00% or more but also to 5.00% or more by using an inorganic filler having transparency and reducing the difference between the refractive index of the polymerizable monomer component and the refractive index of the inorganic filler.

1-2. Regarding Condition [II]

In general, it is known that when light is incident on fine particles having a particle diameter suitable for the curable composition for photofabrication, that is, fine particles having a particle diameter of 0.05 $\mu$m to 5.0 $\mu$m, phenomena such as light shielding, diffraction, Mie scattering, and Rayleigh scattering occur. When Mie scattering and/or Rayleigh scattering occurs, the scattered light spreads not only forward but also laterally and rearward. Then, it is considered that the scattered light spread laterally (side scattered light) reduces the transmittance to the specific activating light set in the condition [I], which causes the generation of cracks, and further reduces the fabrication accuracy of the shaped product.

The spread and intensity of the side scattered light, that is, the intensity distribution of the scattered light in the scattering direction (angle) is affected not only by the particle diameter of the particle, but also by the refractive index of the particle and the polymerizable monomer component present around the particle (serving as dispersion medium). However, in a dispersion system in which particles are dispersed in a polymerizable monomer component as particulate matters having the particle size distribution, particularly in a dispersion system constituted by a plurality of types of particles having different refractive indexes, it is substantially impossible to grasp a scattering behavior of each particle, and it is necessary to grasp a scattering behavior of the entire system. In addition, when the curable composition for photofabrication according to the present disclosure is used for production of a dental prosthesis, as an average "intensity of activating light scattered laterally" of the entire system is larger, the fabrication accuracy is lowered, and the transmittance to the specific activating light is lowered, giving rise to concerns about the generation of cracks due to the increase of the interlayer low-crosslinking-density region. For this reason, in the present disclosure, the combination of the polymerizable monomer component and the inorganic filler used in the curable composition for photofabrication according to the present disclosure is indirectly defined by using the light scattering index Sc (%) defined by the above formula in the base composition (which is uniform, consists of polymerizable monomer component and inorganic filler, polymerizable monomer component and inorganic filler being contained at predetermined mass ratio) serving as a base of the curable composition for photofabrication.

The measurement on the refracted light transmitted through the sample using a variable angle spectrophotometer (goniophotometer) is used, for example, for evaluation on optical properties of a material requiring light diffusibility, such as a cover of a lighting fixture or a projector screen, and is also used for determination on an index for evaluating an optical texture of a tooth substance filling restoration material, specifically, a diffusivity D, as described in Patent Document 4 in the dental field.

In the present disclosure, measurement using a variable angle spectrophotometer for determining the light scattering index Sc can be performed as follows. First, a measurement sample having a thickness of 0.5 mm is prepared in the same manner as in the measurement on transmittance, by using a (measurement) base composition prepared by sampling or separately preparing a part of a (raw material) base composition prepared in the preparation process of the curable composition for photofabrication according to the present disclosure. Next, the measurement sample is set in a three-dimensional variable angle spectrophotometer (for example, GP-200 manufactured by Murakami Color Research Laboratory Co., Ltd.) and is vertically irradiated with "measurement light including light having a specific wavelength λ (nm), including light having a wavelength in a range of $\lambda \pm 50$ (nm) as a main component, and having a maximum intensity in the range" to measure the intensity of transmitted light in each emission angle direction. When light emitted from a light source (capable of emitting light including light having wavelength $\lambda$) attached to the three-dimensional variable angle spectrophotometer does not satisfy the condition related to the measurement light, the measurement light may satisfy the above-described condition using an interference filter (for example, GP-200 manufactured by Murakami Color Research Laboratory Co., Ltd.). The fact that the light having a wavelength in the range of $\lambda \pm 50$ (nm) is the main component means that an integrated value of the intensity of the light having the wavelength of $\lambda \pm 50$ (nm) in a spectrum showing the wavelength distribution (relative spectral distribution) of the measurement light is 90% or more of an integrated value of the intensity of the entire measurement light.

When the light scattering index Sc of the base composition is more than 10%, the formation of the interlayer low-crosslinking-density region, which is a cause of generation of cracks due to the side scattered light, cannot be avoided. From the viewpoint of improving the crack generation prevention effect and the fabrication accuracy, the light scattering index Sc of the base composition is preferably 5.0% or less, and more preferably 3.0% or less. The light scattering index Sc is preferably as low as possible, and a lower limit value thereof is 0.0%.

The light scattering index Sc of the base composition is affected by the blending ratio of the polymerizable monomer component and the inorganic filler, and when the ratio is constant, the light scattering index Sc may be controlled to a certain extent by adjusting the particle size distribution of the inorganic filler, the refractive index (or type) of the particles constituting the inorganic filler, and the refractive index of the entire polymerizable monomer component.

For example, regarding the particle diameter, a particle diameter parameter called "particle size parameter $\alpha$" which is an index of scattering or scattering intensity of light caused by particles is known. When the wavelength (nm) of the incident light is defined as $\lambda_0$, the circumference ratio is defined as $\pi$, and the particle diameter of the inorganic particles is defined as x (nm), the particle diameter parameter $\alpha$ is defined by the formula $\alpha = (\pi \times x)/\lambda_0$. In a case where the particles are transparent to incident light, it is considered that diffraction occurs when the particle size parameter $\alpha$ exceeds 10, and Mie scattering or Reyleigh scattering occurs when the particle size parameter $\alpha$ is 10 or less. When laser light having a wavelength of 405 nm, which is the most common activating light in the vat photopolymerization process, is used, the particle diameter x at which $\alpha = 10$ is obtained is 1,290 nm = 1.29 $\mu$m based on a relation of $x = \alpha \cdot \lambda_0/\pi$, and when laser light having a wavelength of 385 nm is used, the particle diameter x at which $\alpha = 10$ is obtained is 1,226 nm = 1.23 $\mu$m. Since many of the dental inorganic fillers have a particle diameter smaller than the value in many cases, it can be said that the scattering occurs and cracks are likely to be generated.

According to the study by the present inventors, even when Mie scattering or Reyleigh scattering occurs, the light scattering index Sc tends to decrease as the ratio of particles having a particle diameter in which the particle diameter parameter $\alpha$ is in the range of 0.7 to 4, that is, particles satisfying the relation of $x = 0.7 \times (\lambda/\pi)$ to $4 \times (\lambda/\pi)$ (when wavelength of activating light is 405 nm, x = 90 nm to 514 nm, and when wavelength of activating light is 385 nm, x = 86 nm to 490 nm) increases in the particle size distribution measured by the microscopy method using a scanning microscope. For the reason that it is easy to set the light scattering index Sc to 10% or less, the inorganic filler (B) to be used preferably satisfies the following condition 1.

Condition 1: when a particle diameter of each particle constituting the inorganic filler (B) is defined as x (nm) and a circumference ratio is defined as $\pi$, a total number of particles having a particle diameter x (nm) within a range of $0.7 \lambda/\pi$ to $4 \lambda/\pi$ (nm) is 40% or more, preferably 60% or more, and more preferably 80% or more of the number of all particles constituting the inorganic filler (B) in a particle size distribution of the inorganic filler (B) measured by microscopy method using a scanning microscope.

For the reason that the condition [II] is more easily satisfied, in the particle size distribution measured by the microscopy method using the scanning microscope, the ratio of particles having a particle diameter (129 nm to 386 nm when wavelength of activating light is 405 nm) in which the particle diameter parameter $\alpha$ in the inorganic filler (B) is in the range of 1.0 to 3.0 is preferably 50% or more, and more preferably 60% or more, and the ratio of particles having a particle diameter (231 nm to 360 nm when wavelength of activating light is 405 nm) in which the particle diameter parameter $\alpha$ is in the range of 1.8 to 2.8 is preferably 45% or more, and more preferably 50% or more.

Since a generally used photofabrication device (3D printer) uses a light source having an activating light wavelength (peak wavelength) of 380 nm to 420 nm, in order to simultaneously satisfy the conditions [I] and [II], the total number of particles having a particle diameter in the range of 85 nm to 535 nm in the particle size distribution measured by microscopy method using a scanning microscope is preferably 40% or more, more preferably 60% or more, and still more preferably 80% or more of the number of all particles constituting the inorganic filler (B). The range of the particle diameter is more preferably 121 nm to 400 nm, and further preferably 218 nm to 375 nm. That is, in the particle size distribution measured by the microscopy method using a scanning microscope, the total number of particles having a particle diameter in the range of 218 nm to 375 nm is most preferably 80% or more of the number of all particles constituting the inorganic filler (B).

The light scattering index Sc tends to decrease as the difference between the refractive index of the particles and the refractive index of the polymerizable monomer component decreases. In consideration of the refractive index of the inorganic filler and the refractive index of the polymerizable monomer component for dental use, a combination satisfying the following conditions 2 to 4 is preferably adopted as the combination of the inorganic filler and the polymerizable monomer component.

Condition 2: in the inorganic particulate matters constituting the inorganic filler (B), when an inorganic particulate matter consisting of an aggregate of a single type of inorganic particles having a refractive index with respect to a (natrium) line D at 25°C in a range of 1.500 to 1.550 is defined as a specific inorganic particulate matter (b1) and an inorganic particulate matter consisting of an aggregate of a single type of inorganic particles having a refractive index out of the range is defined as a non-specific inorganic particulate matter (b2), the inorganic filler (B)

(1) contains a single type of the specific inorganic particulate matter (b1),
(2) contains a plurality of types of the specific inorganic particulate matters (b1), at least one of the plurality of types of specific inorganic particulate matters (b1) occupying 10 mass% or more of a total mass of the inorganic filler (B), or
(3) contains 90 mass% or more and less than 100 mass% of a single type or a plurality of types of the specific inorganic particulate matters (b1) and more than 0 mass% and less than 10 mass% of a single type or a plurality of types of the non-specific inorganic particulate matters (b2), at least one of the single type or the plurality of types of specific inorganic particulate matters (b1) occupying 10 mass% or more of a total mass of the inorganic filler (B).

Condition 3: a refractive index $n_M$ of the polymerizable monomer component (A) with respect to the line D at 25°C is 1.490 to 1.550.

Condition 4: when a refractive index at which a difference with $n_M$ is largest among the refractive indexes of at least one of the specific inorganic particulate matters (b1) occupying 10 mass® or more of the inorganic filler (B) is defined as $n_F$, an absolute value of a difference between $n_F$ and $n_M$ $|n_F - n_M|$ is 0.035 or less.

When $|n_F - n_M|$ increases, the refraction of the activating light increases at an interface between the inorganic particles and the polymerizable monomer, the intensity of the side scattered light increases, and the light scattering index Sc tends to increase. When the ratio of particles having a particle diameter considerably smaller than the wavelength of the activating light is high, Rayleigh scattering is likely to occur, and the light scattering index Sc tends to increase. Conversely, when the ratio of particles having a particle diameter considerably larger than the wavelength of the activating light is high, the frequency of collision of the activating light with the inorganic particles is significantly increased, and the light scattering index Sc tends to increase.

The refractive index of the inorganic particulate matters made of a single material and the refractive index of the polymerizable monomer component with respect to the line D at 25°C can be measured as follows.

That is, the refractive index of the polymerizable monomer component can be measured using an Abbe refractometer (for example, digital Abbe refractometer DR-A1-PLUS manufactured by Atago Co., Ltd.) by placing the prepared monomer composition on a prism, then viewing a sample from an eyepiece, and reading a value of a display section when an intersection of a boundary line and a cross line is matched (value becoming refractive index). In addition, the refractive index of the inorganic particulate matter is determined by mixing toluene or ethanol and bromonaphthalene to prepare solutions having refractive indexes different from each other by 0.001, then mixing inorganic particulate matters into respective solutions having different refractive indexes, shaking the mixture, and setting the refractive index of the solution observed to be most transparent as the refractive index of the inorganic particulate matter.

1-3. Blending Components of Curable Composition for Photofabrication and Blending Ratio thereof

<Polymerizable Monomer Component>

As the polymerizable monomer component, radically-polymerizable monomers (monomers) can be used without particular limitation. Among the radically-polymerizable monomers, a (meth)acrylate monomer is preferably used because of high curing rate and excellent strength of the obtained fabricated product.

As the (meth)acrylate monomer, a monofunctional (meth)acrylate, a bifunctional (meth)acrylate, or a trifunctional or higher polyfunctional (meth)acrylate may be used. In order to produce a higher strength fabricated product, it is preferable that 50 mass% or more, and preferably 80 mass% or more, based on the total mass of the total radically-polymerizable monomers, is a bifunctional or higher polyfunctional (meth)acrylate. A ratio of the bifunctional or higher polyfunctional (meth)acrylate is more preferably 95 mass% or more.

Examples of the bifunctional or higher polyfunctional (meth)acrylate preferably used include bisphenol A skeleton-containing (meth)acrylates such as 2,2'-bis{4-[3-(meth)acryloyloxy-2-hydroxypropoxy]phenyl}propane, 2,2'-bis[4-(meth)acryloyloxyphenyl]propane, and 2,2'-bis[4-(meth)acryloyloxypolyethoxyphenyl]propane; ethylene glycol (meth)acrylates such as triethylene glycol dimethacrylate and ethylene glycol dimethacrylate; aliphatic di(meth)acrylates such as 1,3-propanediol di(meth)acrylate and 1,9-nonanediol dimethacrylate; urethane group-containing (meth)acrylates such as 1,6-bis(methacryloyloxy-2-ethoxycarbonyl amino)-2,2,4-trimethylhexane; trifunctional (meth)acrylates such as trimethylol propane trimethacrylate; and isocyanate skeleton-containing (meth)acrylates such as tris(2-methacryloyloxyethyl)isocyanurate. Among them, 2,2'-bis[4-(meth)acryloyloxyphenyl]propane, 2,2'-bis[4-(meth)acryloyloxypolyethoxyphenyl]propane, triethylene glycol dimethacrylate, tris(2-methacryloyloxyethyl)isocyanurate, and the like are preferable because of low viscosity and high strength.

Examples of monofunctional (meth)acrylates suitable for use in combination with bifunctional or higher polyfunctional (meth)acrylates include hydroxyethylmethacrylate, methyl(meth)acrylate, ethyl(meth)acrylate, isopropyl(meth)acrylate, hydroxyethyl(meth)acrylate, tetrahydrofurfuryl(meth)acrylate, and glycidyl(meth)acrylate.

These radically-polymerizable monomers may be used alone or in combination of two or more types thereof.

<Inorganic Filler>

In order to increase the mechanical strength such as rigidity of the obtained fabricated product, the curable composition for photofabrication according to the present disclosure contains 40 parts by mass to 400 parts by mass of the inorganic filler containing a single type or a plurality of types of inorganic particulate matters with respect to 100 parts by mass of the polymerizable monomer component. When the content of the inorganic filler is too large, the viscosity of the composition becomes too high. On the other hand, when the content of the inorganic filler is too small, the mechanical strength becomes insufficient. Therefore, the content of the inorganic filler is preferably 50 parts by mass to 350 parts by mass, and more preferably 60 parts by mass to 300 parts by mass with respect to 100 parts by mass of the polymerizable monomer component.

As described above, from the viewpoint of preventing an increase in the viscosity of the composition and preventing precipitation, the inorganic filler needs to contain 80% or more of particles having a particle diameter in the range of 0.05 $\mu$m to 5.0 $\mu$m in the particle size distribution of the inorganic filler measured by microscopy method using a scanning microscope, and a ratio of particles having a particle diameter in the range is preferably 90% or more, and more preferably 95% or more. For the reason of further preventing an increase in the viscosity, a lower limit value of the particle diameter of the particles occupying 80% or more of the inorganic filler is preferably 0.08 $\mu$m, and more preferably 0.1 $\mu$m. For the reason of further preventing precipitation, an upper limit value of the particle diameter of the particles occupying 80% or more of the inorganic filler is preferably 2.0 $\mu$m, and more preferably 1.0 $\mu$m.

As the inorganic filler, under the assumption that the condition [I] is satisfied and the condition [II] is also satisfied as a combination with the polymerizable monomer component, for example, the inorganic particulate matter to be used as the inorganic filler in the tooth restoration material can be used without particular limitation. Examples of the inorganic particulate matter that can be suitably used include particulate matters made of a metal (elemental form); particulate matters made of a metal oxide or a metal composite oxide; particulate matters made of a metal salt such as fluoride, carbonate, sulfate, silicate, hydroxide, chloride, nitrite, or phosphate of a metal; and a mixture of these particulate matters. Particularly preferable examples of the inorganic filler include metal oxides such as amorphous silica, quartz, alumina, zirconia, barium oxide, yttrium oxide, lanthanum oxide, and ytterbium oxide; silica-based composite oxides such as silica-zirconia, silica-titania, silica-titania-barium oxide, and silica-titania-zirconia; glasses such as borosilicate glass, alumino silicate glass, and fluoroalumino silicate glass; metal fluorides such as barium fluoride, strontium fluoride, yttrium fluoride, lanthanum fluoride, and ytterbium fluoride; inorganic carbonates such as calcium carbonate, magnesium carbonate, strontium carbonate, and barium carbonate; and metal sulfates such as magnesium sulfate and barium sulfate. These inorganic fillers may be used by combining a plurality of types of inorganic fillers having different materials, or may be used by combining a plurality of types of inorganic fillers having different particle diameters.

Among them, it is preferable to use amorphous silica, silica-zirconia, silica-titania, silica-titania-barium oxide, silica-titania-zirconia, borosilicate glass, alumino silicate glass, or fluoroaluminosilicate glass, for the reason that the condition [II] is easily satisfied. From the viewpoint of wear resistance of the cured product, it is more preferable to use silica-zirconia.

Furthermore, the inorganic filler may also be blended as a so-called organic-inorganic composite filler.

The inorganic filler is preferably treated with any surface treatment agent represented by a silane coupling agent in order to improve compatibility with the polymerizable monomer component and improve mechanical strength and water resistance. The surface treatment may be performed by a known method. Examples of the silane coupling agent include methyltrimexysilane, methyltriethoxysilane, methyltrichlorosilane, dimethyldichlorosilane, trimethylchlorosilane, vinyltri-chlorosilane, vinyltriethoxysilane, vinyltris($\beta$-methoxyethoxy)silane, $\gamma$-methacryloyloxypropyltrimethoxysilane, methacry-loxyoctyl-8-trimethoxysilane, $\gamma$-chloropropyltrimethoxysilane, $\gamma$-glycidoxypropylmethoxysilane, and hexamethyldisila-zane.

In the case of producing a dental restoration, it is preferable to use particles of silica-zirconia, silica-titania, silica-titania-barium oxide, silica-titania-zirconia, or the like because of strong X-ray contrast properties. From the viewpoint of the wear resistance of the cured product, it is most preferable to use silica-zirconia particles.

<Photopolymerization Initiator>

The photopolymerization initiator needs to have a function of radically polymerizing a polymerizable monomer component by generating a radical with specific activating light including light of a specific wavelength $\lambda$ (nm) in an ultraviolet light range or a visible light range emitted from the light source mounted on the photofabrication device. In other words, the photopolymerization initiator needs to absorb light having a specific wavelength $\lambda$ (nm) to generate radicals. The specific wavelength $\lambda$ may be appropriately determined according to the wavelength of the activating light used in the photofabrication device as long as it is a wavelength in the ultraviolet light range or the visible light range. Examples of the general-purpose photofabrication device include an SLA photofabrication device that emits semiconductor laser light as the activating light, a DLP photofabrication device that emits projector light, and an LCD photofabrication device that emits liquid crystal panel light, and a light source having an activating light wavelength of about 405 nm or about 385 nm is often used. In the present disclosure, the specific wavelength $\lambda$ is preferably 405 nm or 385 nm, and the photofabrication device is preferably SLA, DLP, or LCD.

A content of the photopolymerization initiator may be 0.05 parts by mass to 5.0 parts by mass with respect to 100 parts by mass of the polymerizable monomer component. When the content of the photopolymerization initiator is too large, burrs or the like are generated in the obtained fabricated product, and the accuracy is deteriorated. On the other hand, when the content of the photopolymerization initiator is too small, fabrication cannot be performed in the shaping step. Therefore, the content of the photopolymerization initiator is preferably 0.3 parts by mass to 4.0 parts by mass, and more preferably 0.5 parts by mass to 3.0 parts by mass with respect to 100 parts by mass of the polymerizable monomer component.

As the photopolymerization initiator, a known photopolymerization initiator satisfying the above conditions may be appropriately selected and used. The photopolymerization initiator to be selected is not particularly limited, and examples thereof include self-cleaving photopolymerization initiators, bimolecularly hydrogen-abstracting photopolymerization initiators, photoacid generators, and combinations thereof. The photopolymerization initiators may be used in combination with a photosensitizing dye, an electron-donating compound, or the like.

Examples of self-cleaving photopolymerization initiators that can be suitably used include acylphosphine oxide compounds such as diphenyl(2,4,6-trimethylbenzoyl)-phosphine oxide and phenyl bis(2,4,6-trimethylbenzoyl)-phosphine oxide; benzoketal compounds, benzyne compounds, $\alpha$-aminoacetophenone compounds, $\alpha$-hydroxyacetophe-none compounds, titanocene compounds, and acyloxyme compounds. Examples of the photoacid generators include iodonium salt compounds such as p-isopropylphenyl-p-methylphenyliodonium tetrakispentafluorophenyl borate salt; sulfonium salt compounds such as dimethylphenacylsulfonium hexafluoroantimonate salt; and halomethyl group-substituted triazine compounds such as 2,4,6-tris(trichloromethyl)-s-triazine. Examples of the photosensitizing dye include ketone compounds, coumarin dyes, cyanine dyes, merocyanine dyes, thiazine dyes, azine dyes, acridine dyes, xanthene dyes, squarylium dyes, pyrylium salt dyes, fused polycyclic aromatic compounds (anthracene, perylene, etc.), and thioxanthone compounds. Examples of the electron-donating compound include 4-dimethylaminobenzoic acid ester, 4-dimethylaminotoluene, p-dimethoxybenzene, 1,2,4-trimethoxybenzene, and thiophene compounds.

<Activating Light Absorbent>

When a shaped product such as a dental prosthesis is produced with high accuracy by using the curable composition for photofabrication according to the present disclosure, in order to prevent the activating light emitted from the photofabrication device from being excessively transmitted and the fabrication accuracy of the shaped product from being lowered, it is preferable to contain 0.01 parts by mass to 2.5 parts by mass of an activating light absorbent having a function of absorbing the activating light emitted from the photofabrication device and having no photopolymerization initiation ability with respect to 100 parts by mass of the polymerizable monomer component.

When the content of the activating light absorbent is too large, the light generated from the light source of the photofabrication device in the shaping step does not pass through the composition, and the condition [I] cannot be satisfied. When the content of the activating light absorbent is too small, the accuracy of the obtained fabricated product is lowered. Therefore, the content of the activating light absorbent is preferably 0.04 parts by mass to 2.5 parts by mass, more preferably 0.08 parts by mass to 2.0 parts by mass, and still more preferably 0.25 parts by mass to 1.0 part by mass with respect to 100 parts by mass of the polymerizable monomer component.

The activating light absorbent is not particularly limited as long as it is a compound that absorbs light emitted from the light source mounted on the photofabrication device, and examples thereof include triazole compounds such as 2-(hydroxy-5-methylphenyl)-2H-benzotriazole and 2-(3-tert-butyl-2-hydroxy-5-methylphenyl)-5-chloro-2H-benzotriazole; and benzophenone compounds such as 2,4-dihydroxybenzophenone and 2-hydroxy-4-methoxybenzophenone.

<Other Components>

(Polymerization Inhibitor)

The curable composition for photofabrication according to the present disclosure is preferably blended with a polymerization inhibitor in an amount of 0.01 parts by mass to 5.0 parts by mass with respect to 100 parts by mass of the polymerizable monomer component for the purpose of improving storage stability. When a content of the polymerization inhibitor is too large, the curing of the composition tends to be insufficient in the shaping step, and when the content of the polymerization inhibitor is too small, the storage stability of the composition and the fabrication accuracy tend to decrease. The content of the polymerization inhibitor is preferably 0.01 parts by mass to 5.0 parts by mass, more preferably 0.03 parts by mass to 4.0 parts by mass, and still more preferably 0.05 parts by mass to 2.5 parts by mass with respect to 100 parts by mass of the polymerizable monomer component.

As the polymerization inhibitor, a compound which reacts with the radical generated in the curable composition for photofabrication to deactivate the radical can be used, and examples thereof include di-tert-butyl-p-cresol and 4-methoxyphenol.

(Chain Transfer Agent)

The curable composition for photofabrication according to the present disclosure may be blended with a chain transfer agent in a range of 0.00001 parts by mass to 1.0 part by mass with respect to 100 parts by mass of the polymerizable monomer component for the purpose of reducing shrinkage stress during photofabrication and improving fabrication accuracy. When a content of the chain transfer agent is too large, the polymerization reaction of the curable composition for photofabrication is prevented more than necessary, and when the content of the chain transfer agent is too small, the effect of adding the chain transfer agent cannot be exerted.

Examples of the chain transfer agent include thiol compounds such as butanethiol, thiophenol, mercaptoethanol, octylthiol, lauryl mercaptan; α-alkylstyrene compounds such as 2,4-diphenyl-4-methyl-1-pentene (α-methylstyrene dimer), 2-phenyl-1-propene (α-methylstyrene); and halogenated hydrocarbon substituted with at least one halogen atom such as carbon tetrachloride and ethylene bromide. Among them, an α-alkylstyrene compound, particularly an α-methylstyrene dimer, is preferably used for the reason that the effect of preventing the generation of cracks is high.

(Thermal Polymerization Initiator)

The curable composition for photofabrication according to the present disclosure may be blended with a thermal polymerization initiator as a polymerization initiator for secondary curing. In this case, a thermal polymerization initiator having a 10-hour half-life temperature of 50°C to 130°C is preferably used from the viewpoint of effectively remaining in the stack without functioning during primary curing. Examples of the thermal polymerization initiator that can be suitably used include organic peroxides such as tert-butyl peroxylaurate and benzoyl peroxide; and azo compounds such as azobutylonitrile and azobis(dimethylvaleronitrile).

A content of the thermal polymerization initiator is usually 0.001 parts by mass to 1.0 part by mass, preferably 0.005 parts by mass to 0.3 parts by mass, and more preferably 0.01 parts by mass to 0.1 parts by mass with respect to 100 parts by mass of the polymerizable monomer component.

(Coloring Substance)

The curable composition for photofabrication according to the present disclosure may be blended with a coloring substance in a range in which the condition [I] is satisfied. For example, when the curable composition for photofabrication according to the present disclosure is used to produce a dental restoration such as an inlay, an onlay, a crown, or a denture, it is preferable to blend a coloring substance in order to reproduce a color of a crown or oral mucosa. The coloring substance may be a pigment or a dye. Examples of the pigment include inorganic pigments such as titanium oxide, zinc oxide, zirconium oxide, zinc sulfide, aluminum silicate, calcium silicate, carbon black, iron oxide, copper chromite black, chromium oxide green, chromium green, violet, chromium yellow, lead chromate, lead molybdate, cadmium titanate, nickel titanium yellow, ultramarine blue, cobalt blue, bismuth vanadate, cadmium yellow, and cadmium yellow; and organic pigments such as a monoazo pigment, a diazo pigment, a diazo fused pigment, a perylene pigment, and an anthraquinone pigment.

2. Method for Producing Curable Composition for Photofabrication

In order to produce the curable composition for photofabrication according to the present disclosure, materials exemplified in section 1-3 may be used, and the respective components may be mixed so as to obtain the predetermined composition described above, thereby obtaining a uniform composition in a liquid state, but it is necessary to satisfy the conditions [I] and [II]. In the method for producing a curable composition for photofabrication according to the present disclosure, a combination satisfying specific conditions is used as a combination of a polymerizable monomer component and an inorganic filler, and the curable composition for photofabrication according to the present disclosure can be easily produced by adopting this production method.

That is, the method for producing a curable composition for photofabrication according to the present disclosure includes a mixing step of mixing 100 parts by mass of a polymerizable monomer component (A), 40 parts by mass to 400 parts by mass of an inorganic filler (B) containing a single type or a plurality of types of inorganic particulate matters, 0.01 parts by mass to 5 parts by mass of a photopolymerization initiator (C) having a function of initiating photopolymerization by irradiation with specific activating light, and 0.01 parts by mass to 2.5 parts by mass of an activating light absorbent (D) having a function of absorbing the specific activating light and having no photopolymerization initiation ability, and in the mixing step, the polymerizable monomer (A) and the inorganic filler (B) satisfy the following conditions 1 to 4.

Condition 1: when a particle diameter of each particle constituting the inorganic filler (B) is defined as x (nm) and a circumference ratio is defined as $\pi$, a total number of particles having a particle diameter x (nm) within a range of $0.7 \lambda/\pi$ to $4 \lambda/\pi$ (nm) is 40% or more of the number of all particles constituting the inorganic filler (B) in a particle size distribution of the inorganic filler (B) measured by microscopy method using a scanning microscope.

Condition 2: in the inorganic particulate matters constituting the inorganic filler (B), when an inorganic particulate matter containing an aggregate of a single type of inorganic particles having a refractive index with respect to a D line at 25°C in a range of 1.500 to 1.550 is defined as a specific inorganic particulate matter (b1) and an inorganic particulate matter containing an aggregate of a single type of inorganic particles having a refractive index out of the range is defined as a non-specific inorganic particulate matter (b2), the inorganic filler (B)

(1) contains a single type of the specific inorganic particulate matter (b1),
(2) contains a plurality of types of the specific inorganic particulate matters (b1), at least one of the plurality of types of specific inorganic particulate matters (b1) occupying 10 mass% or more of a total mass of the inorganic filler (B), or
(3) contains 90 mass% or more and less than 100 mass% of a single type or a plurality of types of the specific inorganic particulate matters (b1) and more than 0 mass% and less than 10 mass% of a single type or a plurality of types of the non-specific inorganic particulate matters (b2), at least one of the single type or the plurality of types of specific inorganic particulate matters (b1) occupying 10 mass% or more of a total mass of the inorganic filler (B).

Condition 3: a refractive index $n_M$ of the polymerizable monomer component (A) with respect to the line D at 25°C is 1.490 to 1.550.

Condition 4: when a refractive index at which a difference with $n_M$ is largest among the refractive indexes of at least one of the specific inorganic particulate matters (b1) occupying 10 mass% or more of the inorganic filler (B) is defined as $n_F$, an absolute value of a difference between $n_F$ and $n_M$ $|n_F - n_M|$ is 0.035 or less.

In the method for producing a curable composition for photofabrication according to the present disclosure, a combination matching the criteria is selected from the polymerizable monomer component and the inorganic filler as exemplified in the section 1-3, a photopolymerization initiator having a function of initiating photopolymerization by being irradiated with specific activating light is selected as the photopolymerization initiator, an activating light absorbent absorbing the specific activating light is selected, 100 parts by mass of the polymerizable monomer component, 40 parts by mass to 400 parts by mass of the inorganic filler, 0.01 parts by mass to 5 parts by mass of the photopolymerization initiator, and 0.01 parts by mass to 2.5 parts by mass of the activating light absorbent are weighed and mixed to obtain a uniform liquid composition.

For example, when a curable composition for photofabrication to be used in a general-purpose photofabrication device including a light source that emits semiconductor laser light having a wavelength of 405 nm with a wavelength of 405 nm as a specific wavelength $\lambda$, a particle diameter range of particles occupying 40% or more of the inorganic filler may be set to 90 nm to 514 nm, a photopolymerization initiator that generates radicals by irradiation with light having a wavelength of 405 nm may be selected, and an activating light absorbent that absorbs light having a wavelength of 405 nm may be selected.

Since a general-purpose photofabrication device uses a light source having a wavelength (peak wavelength) of the activating light of 380 nm to 420 nm, a particle diameter range of particles occupying 40% or more (preferably 60% or more, and more preferably 80% or more) of the inorganic filler is set to 85 nm to 535 nm (preferably 121 nm to 400 nm, and more preferably 218 nm to 375 nm), a photopolymerization initiator that generates radicals by irradiation with light having a wavelength of 380 nm to 420 nm may be selected, and an activating light absorbent that absorbs light having a wavelength of 380 nm to 420 nm may be selected.

The components are preferably mixed using a stirrer under light shielding of light for activating the photopolymerization initiator, for example, under red light, until the components become uniform at a room temperature.

The liquid composition thus obtained generally satisfies the conditions [I] and [II], but it is preferable to select the polymerizable monomer component (A) and the inorganic filler (B) used in the mixing step as follows because of higher reliability. That is, it is preferable that the polymerizable monomer component (A) and the inorganic filler (B) satisfying the conditions 1 to 4 are used to separately prepare a base composition containing a composition which consists of the polymerizable monomer component (A) and the inorganic filler (B) and in which a composition ratio of the components is same as a composition ratio of the curable composition for photofabrication as a production target, and the polymerizable monomer component (A) and the inorganic filler (B) found to have a value of a light scattering index Sc (%) of 10% or less are used in the mixing step, the light scattering index being determined by the following formula based on an intensity of transmitted light in a specific emission angle direction obtained by measurement using a variable angle spectrophotometer in which a sample having a thickness of 0.5 mm and containing the base composition is vertically irradiated with measurement light including light having the specific wavelength $\lambda$ (nm), including light having a wavelength in a range of $\lambda \pm 50$ (nm) as a main component, and having a maximum intensity in the range:

$$Sc = \{(I_{70} + I_{75} + I_{80})/(I_0 \times 3)\} \times 100$$

wherein $I_0$, $I_{70}$, $I_{75}$, and $I_{80}$ indicate intensities of the transmitted light in directions of emission angles of 0°, 70°, 75°, and 80°, respectively.

3. Method for producing three-dimensional photofabricated product and method for producing dental restoration according to the present disclosure

In the method for producing a three-dimensional photofabricated product according to the present disclosure, the curable composition for photofabrication according to the present disclosure is used as a liquid photocurable composition to be supplied to a vat of a vat photopolymerization device in a method for producing a three-dimensional fabricated product by the above-described vat photopolymerization process including the shaping step, the washing step, and the secondary curing step. In the method for producing a three-dimensional photofabricated product according to the present disclosure, the curable composition for photofabrication according to the present disclosure is used, and thus a three-dimensional photofabricated product having high mechanical strength and having no cracks on the actual surface can be produced.

In the method for producing a three-dimensional photofabricated product according to the present disclosure, it is preferable that the shaping step includes a first step of irradiating, with activating light, a predetermined position of the liquid photocurable composition held in the vat based on two-dimensional shape data of a height at the first ordinating order and curing the predetermined position, thereby forming a fabricated layer having a shape corresponding to the two-dimensional shape data, and using the "fabricated layer" as a layer to be bonded,

a second step of moving the layer to be bonded upward or downward to supply the liquid photocurable composition directly above or directly below the layer to be bonded in the vat,
a third step of irradiating, with activating light, a predetermined position of the liquid photocurable composition supplied directly above or directly below the layer to be bonded, based on two-dimensional shape data of a height at an order next to the ordinating order in the previous step and curing the predetermined position, thereby forming a new fabricated layer having a shape corresponding to the two-dimensional shape data, and bonding the new fabricated layer to the layer to be bonded to obtain a stack having the new fabricated layer as a new layer to be bonded, and
a fourth step of moving the stack upward or downward to supply the liquid photocurable composition directly above or directly below the new layer to be bonded in the vat,
and that a cycle including the third step and the fourth step is repeated using the new layer to be bonded as the layer to be bonded in the third step, thereby forming a new fabricated layer based on two-dimensional shape data of a height at a final ordinating order in the final third step to obtain a stack.

The vat photopolymerization process including such a shaping step can be suitably performed using a commercially available vat photopolymerization device called a 3D printer.

In the method for producing a three-dimensional photofabricated product according to the present disclosure, after the shaping step, the obtained stack is washed using an organic solvent (subjected to washing step), and then secondarily cured by performing additional activating light irradiation or a heat treatment, or both of the additional activating light irradiation and the heat treatment (subjected to secondary curing step).

Examples of the organic solvent to be used in the washing step include an alcohol solvent such as ethanol, methanol, or isopropyl alcohol; a ketone solvent such as acetone and methyl ethyl ketone; an ether solvent such as diethyl ether, diisopropyl ether, tripropylene glycol monomethyl ether, or tetrahydrofuran; an amide solvent such as N-methylpyrrolidone or dimethylacetamide; and a halogen solvent such as methylene chloride or chloroform. Among them, an alcohol solvent and an ether solvent are preferred because of high washing efficiency and an alcohol solvent is more preferred because of low environmental load.

An irradiation wavelength when additional activating light irradiation is performed in the secondary curing step is not particularly limited as long as activating light having the wavelength is absorbed by a photopolymerization initiator remaining in the stack to generate a radical. An irradiation intensity during the additional activating light irradiation is preferably 5 mW/cm$^2$ or more, more preferably 10 mW/cm$^2$ or more, and further more preferably 30 mW/cm$^2$ or more in order for the photopolymerization initiator remaining in the stack to generate a sufficient amount of radicals. An irradiation time is not particularly limited, and is preferably 1 minute or longer, more preferably 3 minutes or longer, and further preferably 5 minutes or longer. An excessively high irradiation intensity during the additional activating light irradiation leads to excessive heating of the fabricated product, which may cause cracks in the fabricated product. Therefore, the irradiation intensity is preferably 10,000 mW/cm$^2$ or less.

When the curable composition for photofabrication according to the present disclosure contains a thermal polymerization initiator, the thermal polymerization initiator can be used for secondary curing by heating. A heating temperature in this case is preferably 45°C to 120°C, more preferably 50°C to 90°C, and further preferably 55°C to 80°C.

In the method for producing a dental restoration according to the present disclosure, a dental restoration such as an inlay, an onlay, a crown, or a denture is produced by the method for producing a three-dimensional photofabricated product according to the present disclosure. When such a dental restoration is produced, it is preferable to blend an activating light absorbent into the curable composition for photofabrication according to the present disclosure. As three-dimensional shape data indicating a shape of the dental restoration (three-dimensional object) to be used in the shaping step, CAD data designed based on digital data obtained by scanning an oral cavity shape of an individual patient or an oral cavity model prepared for each individual patient may be used. With the method for producing a dental restoration according to the present disclosure, a dental restoration having high mechanical strength and having no cracks on the actual surface can be produced.

EXAMPLES

Hereinafter, the present invention is further specifically described with reference to Examples, but the present invention is not limited to these Examples.

Compounds used in Examples and Comparative Examples and abbreviations thereof are shown below.

(1) Polymerizable Monomer Component (A)

Monomer compositions A1 to A6 prepared by mixing monomer compounds as shown below were used.

(Monomer Compound)

·UDMA: Urethane dimethacrylate
·3G: triethylene glycol dimethacrylate
·D-2.6E: bisphenol A ethylene glycol (EO) adduct dimethacrylate (EO addition number: average 2.6)
·ACMO: acryloylmorpholine

(Monomer Composition)

·Monomer Composition A1:
UDMA: 50 parts by mass, 3G: 20 parts by mass, D-2.6E: 30 parts by mass (refractive index $n_M$: 1.496)
·Monomer Composition A2:
UDMA: 36 parts by mass, 3G: 14 parts by mass, D-2.6E: 50 parts by mass (refractive index $n_M$: 1.508)
•Monomer Composition A3:
UDMA: 29 parts by mass, 3G: 11 parts by mass, D-2.6E: 60 parts by mass (refractive index $n_M$: 1.515)
•Monomer Composition A4:
UDMA: 21.5 parts by mass, 3G: 8.5 parts by mass, D-2.6E: 70 parts by mass (refractive index $n_M$: 1.521)
•Monomer composition A5:
UDMA: 70 parts by mass, 3G: 30 parts by mass (refractive index $n_M$: 1.478)
•Monomer composition A6:
UDMA: 70 parts by mass, ACMO: 30 parts by mass (refractive index $n_M$: 1.491).

The refractive index $n_M$ of each monomer composition is a refractive index to the line D at 25°C. The refractive index $n_M$ was measured by using a digital Abbe refractometer (DR-A1-PLUS, manufactured by Atago Co., Ltd.), placing each of the prepared monomer compositions on a prism, then viewing a sample from an eyepiece, and reading a value of a display portion when an intersection of a boundary line and a cross line was matched.

(2) Inorganic Filler (B)

Inorganic fillers B1 to B8 obtained by mixing inorganic particulate matters shown below as they were or a plurality of types thereof were used so as to obtain the composition shown in Table 1.

·SZ-1: spherical silica-zirconia (γ-methacryloyloxypropyltrimethoxysilane surface-treated product, average primary particle diameter: 280 nm, refractive index $n_F$: 1.522)
·SZ-2: spherical silica-zirconia (γ-methacryloyloxypropyltrimethoxysilane surface-treated product, average primary particle diameter: 150 nm, refractive index $n_F$: 1.522)
·SZ-3: spherical silica-zirconia (γ-methacryloyloxypropyltrimethoxysilane surface-treated product, average primary particle diameter: 450 nm, refractive index $n_F$: 1.540)
·SZ-4: spherical silica-zirconia (γ-methacryloyloxypropyltrimethoxysilane surface-treated product, average primary particle diameter: 80 μm, refractive index $n_F$: 1.523)
·SB-1: amorphous barium glass filler (GM27884 manufactured by Schott AG, γ-methacryloyloxypropyltrimethoxysilane surface-treated product, average primary particle diameter: 700 nm, refractive index $n_F$: 1.530)
·SB-2: amorphous barium glass filler (GM27884 manufactured by Schott AG, γ-methacryloyloxypropyltrimethoxysilane surface-treated product, average primary particle diameter: 200 nm, refractive index $n_F$: 1.530)
·SO-1: spherical silica (γ-methacryloyloxypropyltrimethoxysilane surface-treated product, average primary particle diameter: 1,000 nm, refractive index $n_F$: 1.450)

The average primary particle diameter and the refractive index $n_F$ of each inorganic filler were measured by the method described in the section 1-2.

In addition, a content ratio of particles having a particle diameter of 0.05 μm to 5.0 μm in all primary particles constituting each inorganic filler was measured based on the particle size distribution obtained by microscopy method using a scanning microscope (represented as "content ratio of specific particle" in Table 1). Furthermore, regarding the inorganic filler used in each of Examples and Comparative Examples, a content ratio of particles having a (primary) particle diameter in which the particle diameter parameter α (represented as "content ratio of α-satisfied particle" in Table 1) is within a specific range was determined based on the particle size distribution determined by microscopy method using a scanning microscope. Specifically, a content ratio R1 (%) of particles having the particle diameter parameter α in the range of 0.7 to 4, a content ratio R2 (%) of particles having the particle diameter parameter α in the range of 1.0 to 3.0, and a content ratio R3 (%) of particles having the particle diameter parameter α in the range of 1.8 to 2.8 were obtained. Results are also shown in Table 1. The particle size distribution was obtained by measuring the number n (50 or more) of all primary particles observed in a unit field of view of a scanning electron microscope photograph of each inorganic filler and the particle diameter (maximum diameter) $X_i$ (nm) of all primary particles.

[Table 1]

| | Inorganic filler (B) | | | | |
|---|---|---|---|---|---|
| | Composition of inorganic particulate matter | Content ratio of specific particle (%) | Content ratio of α-satisfied particle | | |
| Symbol | Inorganic particulate matter (Refractive index: $n_F$, average particle diameter (nm): X) :Content ratio (Mass%) | | α = 0.7-4.0 R1 (%) | α = 1.0-3.0 R2 (%) | α = 1.8-2.8 R3 (%) |
| B1 | SZ-1($n_F$ = 1.522, X = 280):100 | 100 | 100 | 100 | 100 |
| B2 | SZ-2($n_F$ = 1.522, X = 150):100 | 100 | 100 | 80 | 30 |
| B3 | SZ-3($n_F$ = 1.540, X = 450):100 | 100 | 95 | 50 | 20 |
| B4 | SZ-1($n_F$ = 1.522, X = 280):80 SB-1($n_F$ = 1.530, X = 700):20 | 100 | 90 | 85 | 83 |
| B5 | SB-1($n_F$ = 1.530, X = 700):80 SB-2($n_F$ = 1.530, X = 200):20 | 100 | 45 | 30 | 20 |

(continued)

| Inorganic filler (B) | | | | | | |
|---|---|---|---|---|---|---|
| | Composition of inorganic particulate matter | | Content ratio of specific particle (%) | Content ratio of α-satisfied particle | | |
| Symbol | Inorganic particulate matter (Refractive index: $n_F$, average particle diameter (nm): X) :Content ratio (Mass%) | | | $\alpha$ = 0.7-4.0 R1 (%) | $\alpha$ = 1.0-3.0 R2 (%) | $\alpha$ = 1.8-2.8 R3 (%) |
| B6 | SZ-3($n_F$ = 1.540, X = 450):70 SZ-1 ($n_F$ = 1.522, X= 280):30 | | 100 | 97 | 50 | 40 |
| B7 | SZ-1 ($n_F$ = 1.522, X = 280):70 SO-1($n_F$ = 1.450, X = 1000):30 | | 100 | 98 | 95 | 92 |
| B8 | SO-1($n_F$ = 1.450, X=1000):100 | | 100 | 0 | 0 | 0 |

(3) Photopolymerization Initiator

·BAPO: phenyl bis (2,4, 6-trimethylbenzoyl) phosphine oxide (radicals are generated by irradiation with activating light of 405 nm)

·TPO: 2,4,6-trimethylbenzoyl-diphenylphosphine oxide

(4) Activating Light Absorbent

·SS3: 2-(3-tert-butyl-2-hydroxy-5-methylphenyl)-5-chloro-2H-benzotriazole

(5) Polymerization Inhibitor

·BHT: dibutylhydroxytoluene

·HQME: hydroquinone methyl ether

(6) Coloring Substance

·Titanium oxide (average primary particle diameter: 200 $\mu$m)

<Example 1>

(1) Preparation of Base Composition for Sc Evaluation and Curable Composition for Photofabrication

To 100 parts by mass of a polymerizable monomer component containing a monomer composition A1 (50 parts by mass of UDMA, 20 parts by mass of 3G, 30 parts by mass of D-2.6E), 150 parts by mass of SZ-1 as an inorganic filler was added, and the mixture was stirred and mixed until the mixture became uniform, followed by defoaming to prepare a base composition for Sc evaluation.
In addition, a base composition for raw materials is separately prepared in the same manner, 1.4 parts by mass of BAPO as a photopolymerization initiator, 0.7 parts by mass of SS3 as an activating light absorbent, 0.1 parts by mass of HQME as a polymerization inhibitor, and 0.1 parts by mass of BHT as a polymerization inhibitor (blending amounts are blending amounts with respect to 100 parts by mass of polymerizable monomer component) were added, and the mixture was stirred and mixed under red light until the mixture became uniform, followed by defoaming to prepare a liquid curable composition for photofabrication.

(2) Measurement on Light Scattering Index (Sc)

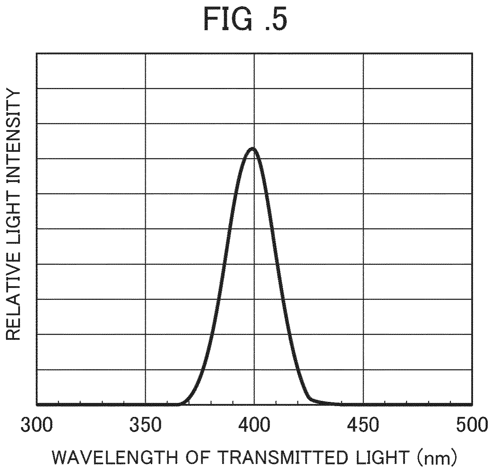

The base composition for Sc evaluation was filled in a mold (25 mm × 25 mm × thickness of 0.5 mm) made of a fluororesin. Upper and lower surfaces were pressed with a slide glass to adjust the thickness of the composition to 0.5 mm, and then the slide glass was removed to prepare an evaluation sample. This evaluation sample was set in a three-dimensional variable angle spectrophotometer (GP-200, manufactured by Murakami Color Research Laboratory Co., Ltd.), and was vertically irradiated with measurement light to measure a luminous intensity distribution of transmitted light. In the measurement, an interference filter (GP-200 manufactured by Murakami Color Technology Research Co., Ltd.) was installed between a light source of the three-dimensional variable angle spectrophotometer and the evaluation sample, and light having a wavelength distribution (relative spectral distribution) illustrated in FIG. 5 was used as measurement light. The light scattering index (Sc) was calculated in accordance with the following formula based on the obtained luminous intensity distribution.

$$Sc = [(I_{70} + I_{75} + I_{80})/I_0 \times 3] \times 100$$

As a result, the light scattering index Sc was 0.1 (%).

(3) Measurement on Transmittance to Activating Light

The curable composition for photofabrication obtained in (1) above was filled in a mold (25 mm × 25 mm × thickness of 0.5 mm) made of a fluororesin. Upper and lower surfaces were pressed with a slide glass to adjust the thickness of the composition to 0.5 mm, and then the slide glass was removed to prepare a resin composition having a thickness of 0.5 mm. Regarding the resin composition, the transmittance to light having a wavelength of 405 nm was measured using a color difference meter (spectrophotometer SE7700 manufactured by NIPPON DENSHOKU KOGYO CO., LTD.). As a result, the transmittance was 5.30%.

(4) Production of Three-Dimensional Photofabricated Product

The curable composition for photofabrication obtained in (1) above was supplied to a resin tray (vat) of a 3D printer (DW029D manufactured by DWS, wavelength: 405 nm, irradiation intensity: 83 mW), and a shaping step was performed using stereolithography data (hereinafter abbreviated as "stl data") of a rectangular parallelepiped shape of 10 mm × 10 mm × 25 mm to prepare a shaped product (stack) having a stacked structure (made of curable composition for photofabrication). Next, the resulting shaped product was then immersed in a plastic container filled with ethanol for 15 minutes, shaken gently, dried, and then subjected to additional light irradiation (secondary curing) for 30 minutes using a UV CURING UNIT UVIS-2 (manufactured by DWS) to produce a three-dimensional photofabricated product.

(5) Evaluation of Three-Dimensional Photofabricated Product

The three-dimensional photofabricated product obtained in (4) above was used as an evaluation sample for fabrication accuracy evaluation, crack evaluation, and 3-point bending fracture strength evaluation. Evaluation methods and evaluation criteria are described below.

(Fabrication Accuracy Evaluation)

A length of each side of the evaluation sample was measured in 0.01 mm increments, a value ("measured value/stl data set value") obtained by dividing a measured value by a set value (length of each side set in stl data) was calculated for each side, and an average value thereof was evaluated as an average value of fabrication accuracy. As a result, the average value of the fabrication accuracy was "1.01".

(Crack Evaluation)

A surface of the evaluation sample was observed with an optical microscope (50 times), the evaluation sample was coated with platinum to a thickness of 5 nm and then observed with a scanning microscope (1,000 times) to check for the number of cracks observed on one surface of 10 mm × 25 mm in the evaluation sample and the crack width, and evaluation was performed according to the following evaluation criteria. As a result, the evaluation was "A1".

-Evaluation Criteria-

A0: No cracks are observed on the surface of the cured product.

A1: The number of cracks observed on the surface of the cured product is 5 or less, and each crack width is 10 $\mu$m or less, which is within an allowable range.

A2: The number of cracks observed on the surface of the cured product is 10 or less, and each crack width is 10 $\mu$m or less, which is within an allowable range.

A3: The number of cracks observed on the surface of the cured product is 20 or less, and each crack width is 10 $\mu$m or less, which is within an allowable range.

B: The number of cracks observed on the surface of the cured product is 20 or less, and the crack width is 10 $\mu$m to 40 $\mu$m.

C: 20 or more cracks having a crack width of 10 $\mu$m to 40 $\mu$m are observed on the surface of the cured product.

D: A large amount of cracks having a crack width of more than 40 $\mu$m are observed on the surface of the cured product.

<Examples 2 to 14 and Comparative Examples 1 to 8>

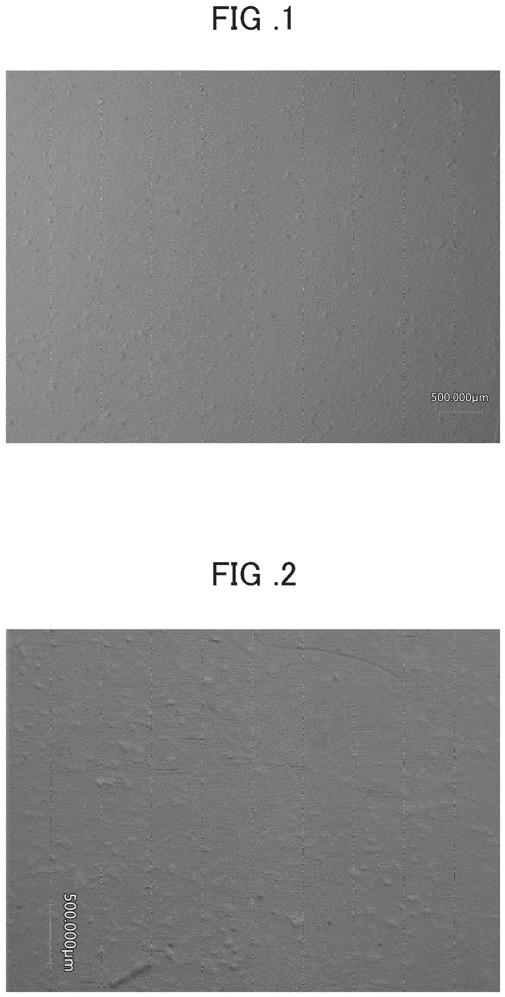

Base compositions for Sc evaluation and curable compositions for photofabrication were prepared in the same manner as in Example 1 except that components and blending amounts thereof used in preparing the base composition for Sc evaluation and the curable composition for photofabrication in Example 1 were changed as shown in Tables 2 and 3. Thereafter, each of the obtained compositions was evaluated in the same manner as in Example 1, and a three-dimensional photofabricated product using the obtained curable composition for photofabrication was produced and evaluated in the same manner as in Example 1. The evaluation results are shown in Tables 4 and 5. In addition, optical microscope images (magnification of 50 times) of Example 7 (evaluated as A0), Example 11 (evaluated as A2), and Comparative Example 1 (evaluated as D), which were taken during observation at the time of crack evaluation, are shown in FIGS. 1, 2, and 3, respectively.

<Comparative Example 9>

A curable composition for photofabrication was prepared according to the method described in Example 1 of Patent Document 3. Specifically, the polymerizable monomer component (A) and the inorganic filler (B) of the types shown in Table 3 were used in the blending ratios shown in Table 3, and 3.0 parts by mass of TPO as a polymerization initiator and 0.05 parts by mass of BHT as a polymerization inhibitor (blending amounts thereof are each blending amount with respect to 100 parts by mass of polymerizable monomer component) were added to prepare a curable composition for photofabrication. The three-dimensional photofabricated product using the obtained curable composition for photofabrication was produced and evaluated in the same manner as in Example 1. A base composition for Sc evaluation was prepared in the same manner as in Example 1, and each composition was evaluated in the same manner as in Example 1. The evaluation results are shown in Table 5. In addition, optical microscope images (magnification of 50 times) taken during observation at the time of crack evaluation in Comparative Example 9 (evaluated as C) are shown in FIG. 4.

[Table 2]

| No. | | Curable composition for photofabrication Abbreviation of component (Part by mass) | | | | | |
|---|---|---|---|---|---|---|---|
| | | Polymerizable monomer component (A) | Inorganic filler (B) | Photopolymerization initiator (C) | Activating light absorbent (D) | Other components polymerization inhibitor | Other components coloring substance |
| Example | 1 | A1(100) | B1(150) | BAPO (1. 4) | SS3 (0.7) | BHT (0.1) HQME (0.1) | - |
| | 2 | A1 (100) | B1 (100) | BAPO (1. 4) | SS3 (0.7) | BHT (0.1) HQME (0.1) | - |
| | 3 | A1 (100) | B1 (67) | BAPO (1. 4) | SS3 (0.7) | BHT (0.1) HQME (0.1) | - |
| | 4 | A2 (100) | B1 (150) | BAPO (1. 4) | SS3 (0.7) | BHT (0.1) HQME (0.1) | - |
| | 5 | A3 (100) | B1 (150) | BAPO (1. 4) | SS3 (0.7) | BHT (0.1) HQME (0.1) | - |
| | 6 | A4 (100) | B1 (150) | BAPO (1. 4) | SS3 (0.7) | BHT (0.1) HQME (0.1) | - |
| | 7 | A4 (100) | B1 (150) | BAPO (1. 4) | SS3 (0.3) | BHT (0.1) HQME (0.1) | - |
| | 8 | A4 (100) | B1 (150) | BAPO (1. 4) | SS3 (0.1) | BHT (0.1) HQME (0.1) | - |
| | 9 | A4 (101) | B1 (150) | BAPO (1. 4) | - | BHT (0.1) HQME (0.2) | - |
| | 10 | A4 (100) | B1 (150) | BAPO (1. 4) | SS3 (0.7) | BHT (0.1) HQME (0.1) | Titanium oxide(0.02) |
| | 11 | A4 (100) | B1 (150) | BAPO (1. 4) | SS3 (0.7) | BHT (0.1) HQME (0.1) | Titanium oxide(0.04) |
| | 12 | A1 (100) | B2 (150) | BAPO (1. 4) | SS3 (0.7) | BHT (0.1) HQME (0.1) | - |
| | 13 | A1 (100) | B4 (150) | BAPO (1. 4) | SS3 (0.7) | BHT (0.1) HQME (0.1) | - |
| | 14 | A1 (100) | B5 (150) | BAPO (1. 4) | SS3 (0.7) | BHT (0.1) HQME (0.1) | - |

[Table 3]

| No. | | Curable composition for photofabrication Abbreviation of component (Part by mass) | | | | | |
|---|---|---|---|---|---|---|---|
| | | Polymerizable monomer component (A) | Inorganic filler (B) | Photopolymerization initiator (C) | Activating light absorbent (D) | Other components polymerization inhibitor | Other components coloring substance |
| Comparative example | 1 | A1 (100) | B3 (150) | BAPO (1.4) | SS3 (0.7) | BHT (0.1) HQME (0.1) | - |
| | 2 | A1 (100) | B3 (150) | BAPO (1.4) | SS3 (0.3) | BHT (0.1) HQME (0.1) | - |
| | 3 | A1 (100) | B3 (150) | BAPO (1.4) | SS3 (0.1) | BHT (0.1) HQME (0.1) | - |
| | 4 | A4 (100) | B6 (150) | BAPO (1.4) | SS3 (0.7) | BHT (0.1) HQME (0.1) | - |
| | 5 | A1 (100) | B7 (150) | BAPO (1.4) | SS3 (0.7) | BHT (0.1) HQME (0.1) | - |
| | 6 | A5 (100) | B1 (150) | BAPO (1.4) | SS3 (0.7) | BHT (0.1) HQME (0.1) | - |
| | 7 | A5 (100) | B2 (150) | BAPO (1.4) | SS3 (0.7) | BHT (0.1) HQME (0.1) | - |
| | 8 | A1 (100) | B3 (67) | BAPO (1.4) | SS3 (0.7) | BHT (0.1) HQME (0.1) | - |
| | 9 | A6 (100) | B8 (100) | TPO (3.0) | - | BHT (0.05) | - |

[Table 4]

| No. | | Difference in refractive indexes of (A) and (B) |nF-nM| | Transmittance to activating light (405 nm) (%) | Light scattering index Sc of base composition (%) | Evaluation results of three-dimensional photofabricated product | |
|---|---|---|---|---|---|---|
| | | | | | Fabrication accuracy | Crack evaluation |
| Example | 1 | 0.026 | 5.30 | 0.1 | 1.01 | A1 |
| | 2 | 0.026 | 5.51 | 0.1 | 1.01 | A1 |
| | 3 | 0.026 | 5.72 | 0 | 1.01 | A1 |
| | 4 | 0.007 | 9.44 | 0 | 1.01 | A1 |
| | 5 | 0.014 | 6.97 | 0 | 1.01 | A1 |
| | 6 | 0.001 | 14.20 | 0 | 1.01 | A1 |
| | 7 | 0.001 | 18.77 | 0 | 1.02 | A0 |
| | 8 | 0.001 | 25.10 | 0 | 1.05 | A0 |
| | 9 | 0.001 | 30.20 | 0 | 1.20 | A0 |
| | 10 | 0.001 | 8.20 | 0.2 | 1.01 | A2 |
| | 11 | 0.001 | 5.50 | 0.4 | 1.01 | A2 |
| | 12 | 0.026 | 3.14 | 2.9 | 1.02 | A3 |
| | 13 | 0.034 | 3.10 | 0.1 | 1.02 | A3 |
| | 14 | 0.034 | 2.60 | 0.1 | 1.02 | A3 |

[Table 5]

| No. | | Difference in refractive indexes of (A) and (B) \|nF-nM\| | Transmittance to activating light (405 nm) (%) | Light scattering index Sc of base composition (%) | Evaluation results of three-dimensional photofabricated product | |
|---|---|---|---|---|---|---|
| | | | | | Fabrication accuracy | Crack evaluation |
| Comparat ive example | 1 | 0.040 | 0.21 | 26 | 1.06 | D |
| | 2 | 0.040 | 0.43 | 26 | 1.20 | D |
| | 3 | 0.040 | 0.90 | 26 | 1.50 | B |
| | 4 | 0.019 | 0.49 | 9.3 | 1.05 | C |
| | 5 | 0.046 | 0.51 | 1.1 | 1.02 | D |
| | 6 | 0.044 | 0.44 | 0.7 | 1.02 | D |
| | 7 | 0.044 | 0.33 | 4.2 | 1.02 | D |
| | 8 | 0.040 | 0.97 | 12.2 | 1.05 | C |
| | 9 | 0.409 | 0.50 | 15.2 | 1.01 | C |

As shown in Table 4, since the curable compositions for photofabrication of Examples 1 to 14 have a small light scattering index Sc and a high activating light transmittance, the fabrication accuracy is high and the generation of cracks is prevented. On the other hand, as shown in Table 5, since the curable compositions for photofabrication of Comparative Examples 1 to 3 have a large light scattering index Sc and a small activating light transmittance, the fabrication accuracy is low and cracks are generated. Since the curable compositions for photofabrication of Comparative Examples 4 to 7 have a small light scattering index Sc and a small activating light transmittance, cracks are generated. Since the curable composition for photofabrication of Comparative Example 8 has a large light scattering index Sc, the fabrication accuracy is low and cracks are generated. The curable composition for photofabrication of Comparative Example 9 is prepared according to the method described in Patent Literature 3, since the activating light transmittance is small, cracks are generated.

## Claims

1. A curable composition for three-dimensional photofabrication to be used as a liquid photocurable composition held in a vat in a vat photopolymerization process for producing a three-dimensional photofabricated product by irradiating a predetermined position of the liquid photocurable composition with activating light including light having a specific wavelength $\lambda$ (nm) in an ultraviolet light range or a visible light range and selectively curing the liquid photocurable composition present at the position, the curable composition comprising:

   100 parts by mass of a polymerizable monomer component (A); 40 parts by mass to 400 parts by mass of an inorganic filler (B) containing a single type or a plurality of types of inorganic particulate matters; and 0.01 parts by mass to 5 parts by mass of a photopolymerization initiator (C) having a function of initiating photopolymerization by irradiation with the activating light,
   80% or more of all primary particles constituting the inorganic filler (B) being particles having a particle diameter of 0.05 $\mu$m to 5.0 $\mu$m in a particle size distribution of the inorganic filler (B) measured by microscopy method using a scanning microscope,
   a transmittance to the activating light measured for a sample having a thickness of 0.5 mm and containing the curable composition for three-dimensional photofabrication being 1.00% to 50.00%.

2. The curable composition for three-dimensional photofabrication according to claim 1, further comprising:
   0.01 parts by mass to 2.5 parts by mass of an activating light absorbent (D) having a function of absorbing the activating light and having no photopolymerization initiation ability, wherein

   the polymerizable monomer component (A) and the inorganic filler (B) have a value of a light scattering index Sc (%) of 10 (%) or less,

the light scattering index Sc (%) being determined by the following formula based on an intensity of transmitted light in a specific emission angle direction obtained by measurement using a variable angle spectrophotometer in which a sample having a thickness of 0.5 mm and containing a base composition is vertically irradiated with measurement light including light having the specific wavelength $\lambda$ (nm), including light having a wavelength in a range of $\lambda \pm 50$ (nm) as a main component, and having a maximum intensity in the range, the base composition being a composition consisting of the polymerizable monomer component (A) and the inorganic filler (B) and having a composition ratio of the components same as a composition ratio of the curable composition for three-dimensional photofabrication,

$$Sc = \{(I_{70} + I_{75} + I_{80})/(I_0 \times 3)\} \times 100$$

wherein $I_0$, $I_{70}$, $I_{75}$, and $I_{80}$ indicate intensities of the transmitted light in directions of emission angles of 0°, 70°, 75°, and 80°, respectively.

3. The curable composition for three-dimensional photofabrication according to claim 1 or 2, wherein when a particle diameter of each particle constituting the inorganic filler (B) is defined as x (nm) and a circumference ratio is defined as $\pi$, a total number of particles having a particle diameter x (nm) within a range of $0.7\,\lambda/\pi$ to $4\,\lambda/\pi$ (nm) is 40% or more of the number of all particles constituting the inorganic filler (B) in a particle size distribution of the inorganic filler (B) measured by microscopy method using a scanning microscope.

4. The curable composition for three-dimensional photofabrication according to claim 2, wherein in the inorganic particulate matters constituting the inorganic filler (B), when an inorganic particulate matter consisting of an aggregate of a single type of inorganic particles having a refractive index with respect to a line D at 25°C in a range of 1.500 to 1.550 is defined as a specific inorganic particulate matter (b1) and an inorganic particulate matter consisting of an aggregate of a single type of inorganic particles having a refractive index out of the range is defined as a non-specific inorganic particulate matter (b2), the inorganic filler (B)

(1) contains a single type of the specific inorganic particulate matter (b1),
(2) contains a plurality of types of the specific inorganic particulate matters (b1), at least one of the plurality of types of specific inorganic particulate matters (b1) occupying 10 mass% or more of a total mass of the inorganic filler (B), or
(3) contains 90 mass% or more and less than 100 mass% of a single type or a plurality of types of the specific inorganic particulate matters (b1) and more than 0 mass% and less than 10 mass% of a single type or a plurality of types of the non-specific inorganic particulate matters (b2), at least one of the single type or the plurality of types of specific inorganic particulate matters (b1) occupying 10 mass% or more of a total mass of the inorganic filler (B), a refractive index $n_M$ of the polymerizable monomer component (A) with respect to the line D at 25°C is 1.490 to 1.550, and
when a refractive index at which a difference with $n_M$ is largest among the refractive indexes of at least one of the specific inorganic particulate matters (b1) occupying 10 mass% or more of the inorganic filler (B) is defined as $n_F$, an absolute value of a difference between $n_F$ and $n_M$ $|n_F - n_M|$ is 0.035 or less.

5. A method for producing a curable composition for three-dimensional photofabrication, comprising:

a mixing step of mixing 100 parts by mass of a polymerizable monomer component (A), 40 parts by mass to 400 parts by mass of an inorganic filler (B) containing a single type or a plurality of types of inorganic particulate matters, 0.01 parts by mass to 5 parts by mass of a photopolymerization initiator (C) having a function of initiating photopolymerization by irradiation with activating light including light having a specific wavelength $\lambda$ (nm) in an ultraviolet light range or a visible light range, and 0.01 parts by mass to 2.5 parts by mass of an activating light absorbent (D) having a function of absorbing the activating light and having no photopolymerization initiation ability,
wherein, in the mixing step, the polymerizable monomer (A) and the inorganic filler (B) satisfy the following conditions 1 to 4:

condition 1: when a particle diameter of each particle constituting the inorganic filler (B) is defined as x (nm) and a circumference ratio is defined as $\pi$, a total number of particles having a particle diameter x (nm) within a range of $0.7\,\lambda/\pi$ to $4\,\lambda/\pi$ (nm) is 40% or more of the number of all particles constituting the inorganic filler (B) in a particle size distribution of the inorganic filler (B) measured by microscopy method using a scanning microscope;

condition 2: in the inorganic particulate matters constituting the inorganic filler (B), when an inorganic particulate matter consisting of an aggregate of a single type of inorganic particles having a refractive index with respect to a line D at 25°C in a range of 1.500 to 1.550 is defined as a specific inorganic particulate matter (b1) and an inorganic particulate matter consisting of an aggregate of a single type of inorganic particles having a refractive index out of the range is defined as a non-specific inorganic particulate matter (b2), the inorganic filler (B)

> (1) contains a single type of the specific inorganic particulate matter (b1),
> (2) contains a plurality of types of the specific inorganic particulate matters (b1), at least one of the plurality of types of specific inorganic particulate matters (b1) occupying 10 mass% or more of a total mass of the inorganic filler (B), or
> (3) contains 90 mass% or more and less than 100 mass% of a single type or a plurality of types of the specific inorganic particulate matters (b1) and more than 0 mass% and less than 10 mass% of a single type or a plurality of types of the non-specific inorganic particulate matters (b2), at least one of the single type or the plurality of types of specific inorganic particulate matters (b1) occupying 10 mass% or more of a total mass of the inorganic filler (B);

> condition 3: a refractive index $n_M$ of the polymerizable monomer component (A) with respect to the line D at 25°C is 1.490 to 1.550; and
> condition 4: when a refractive index at which a difference with $n_M$ is largest among the refractive indexes of at least one of the specific inorganic particulate matters (b1) occupying 10 mass® or more of the inorganic filler (B) is defined as $n_F$, an absolute value of a difference between $n_F$ and $n_M$ $|n_F - n_M|$ is 0.035 or less.

6. The method for producing a curable composition for three-dimensional photofabrication according to claim 5, wherein the polymerizable monomer component (A) and the inorganic filler (B) satisfying the conditions 1 to 4 are used to separately prepare a base composition containing a composition which consists of the polymerizable monomer component (A) and the inorganic filler (B) and in which a composition ratio of the components is same as a composition ratio of the curable composition for three-dimensional photofabrication as a production target, and

> the polymerizable monomer component (A) and the inorganic filler (B) found to have a value of a light scattering index Sc (%) of 10% or more are used in the mixing step, the light scattering index being determined by the following formula based on an intensity of transmitted light in a specific emission angle direction obtained by measurement using a variable angle spectrophotometer in which a sample having a thickness of 0.5 mm and containing the base composition is vertically irradiated with measurement light including light having the specific wavelength $\lambda$ (nm), including light having a wavelength in a range of $\lambda \pm 50$ (nm) as a main component, and having a maximum intensity in the range:

$$Sc = \{(I_{70} + I_{75} + I_{80})/(I_0 \times 3)\} \times 100$$

> wherein $I_0$, $I_{70}$, $I_{75}$, and $I_{80}$ indicate intensities of the transmitted light in directions of emission angles of 0°, 70°, 75°, and 80°, respectively.

7. A method for producing a three-dimensional photofabricated product, which is a method for producing a three-dimensional photofabricated product by irradiating a predetermined position of a liquid photocurable composition held in a vat with activating light including light having a specific wavelength $\lambda$ (nm) in an ultraviolet light range or a visible light range and selectively curing the liquid photocurable composition present at the position, the method comprising:

> a shaping step of digitizing and ordinating a height direction of a three-dimensional object based on three-dimensional shape data indicating a shape of the three-dimensional object, generating two-dimensional shape data indicating a cross-sectional shape of the three-dimensional object at each ordinated height, irradiating the liquid photocurable composition held in the vat with the activating light at a predetermined position determined in advance based on the two-dimensional shape data, thereby selectively primarily curing the liquid photocurable composition present at the position, forming a fabricated layer having the cross-sectional shape, and sequentially forming and stacking the fabricated layer having the cross-sectional shape at each height according to an order of the ordinating, to obtain a stack having a shape corresponding to the shape of the three-dimensional object;
> a washing step of washing, with an organic solvent, the stack obtained in the shaping step; and
> a secondary curing step of secondarily curing the stack washed in the washing step by performing additional activating light irradiation or a heating treatment, or both of the additional activating light irradiation and the heating treatment,
the curable composition for three-dimensional photofabrication according to claim 1 being used as the liquid photocurable composition.

8. A method for producing a dental restoration, comprising: producing the dental restoration by the method for producing a three-dimensional photofabricated product according to claim 7.

**Amended claims under Art. 19.1 PCT**

1. A curable composition for three-dimensional photofabrication to be used as a liquid photocurable composition held in a vat in a vat photopolymerization process for producing a three-dimensional photofabricated product by irradiating a predetermined position of the liquid photocurable composition with activating light including light having a specific wavelength $\lambda$ (nm) in an ultraviolet light range or a visible light range and selectively curing the liquid photocurable composition present at the position, the curable composition comprising:

100 parts by mass of a polymerizable monomer component (A); 40 parts by mass to 400 parts by mass of an inorganic filler (B) containing a single type or a plurality of types of inorganic particulate matters; 0.01 parts by mass to 5 parts by mass of a photopolymerization initiator (C) having a function of initiating photopolymerization by irradiation with the activating light, and 0.01 parts by mass to 2.5 parts by mass of an activating light absorbent (D) having a function of absorbing the activating light and having no photopolymerization initiation ability,
80% or more of all primary particles constituting the inorganic filler (B) being particles having a particle diameter of 0.05 $\mu$m to 5.0 $\mu$m in a particle size distribution of the inorganic filler (B) measured by microscopy method using a scanning microscope,
a transmittance to the activating light measured for a sample having a thickness of 0.5 mm and containing the curable composition for three-dimensional photofabrication being 1.00% to 50.00%,
the polymerizable monomer component (A) and the inorganic filler (B) have a value of a light scattering index Sc (%) of 10 (%) or less,
the light scattering index Sc (%) being determined by the following formula based on an intensity of transmitted light in a specific emission angle direction obtained by measurement using a variable angle spectrophotometer in which a sample having a thickness of 0.5 mm and containing a base composition is vertically irradiated with measurement light including light having the specific wavelength $\lambda$ (nm), including light having a wavelength in a range of $\lambda \pm 50$ (nm) as a main component, and having a maximum intensity in the range, the base composition being a composition consisting of the polymerizable monomer component (A) and the inorganic filler (B) and having a composition ratio of the components same as a composition ratio of the curable composition for three-dimensional photofabrication,

$$Sc = \{(I_{70} + I_{75} + I_{80})/(I_0 \times 3)\} \times 100$$

wherein $I_0$, $I_{70}$, $I_{75}$, and $I_{80}$ indicate intensities of the transmitted light in directions of emission angles of 0°, 70°, 75°, and 80°, respectively.

2. The curable composition for three-dimensional photofabrication according to claim 1, wherein when a particle diameter of each particle constituting the inorganic filler (B) is defined as x (nm) and a circumference ratio is defined as $\pi$, a total number of particles having a particle diameter x (nm) within a range of 0.7 $\lambda/\pi$ to 4 $\lambda/\pi$ (nm) is 40% or more of the number of all particles constituting the inorganic filler (B) in a particle size distribution of the inorganic filler (B) measured by microscopy method using a scanning microscope.

3. The curable composition for three-dimensional photofabrication according to claim 1, wherein in the inorganic particulate matters constituting the inorganic filler (B), when an inorganic particulate matter consisting of an aggregate of a single type of inorganic particles having a refractive index with respect to a line D at 25°C in a range of 1.500 to 1.550 is defined as a specific inorganic particulate matter (b1) and an inorganic particulate matter consisting of an aggregate of a single type of inorganic particles having a refractive index out of the range is defined as a non-specific inorganic particulate matter (b2), the inorganic filler (B)

(1) contains a single type of the specific inorganic particulate matter (b1),
(2) contains a plurality of types of the specific inorganic particulate matters (b1), at least one of the plurality of types of specific inorganic particulate matters (b1) occupying 10 mass% or more of a total mass of the inorganic filler (B), or

(3) contains 90 mass% or more and less than 100 mass% of a single type or a plurality of types of the specific inorganic particulate matters (b1) and more than 0 mass% and less than 10 mass% of a single type or a plurality of types of the non-specific inorganic particulate matters (b2), at least one of the single type or the plurality of types of specific inorganic particulate matters (b1) occupying 10 mass% or more of a total mass of the inorganic filler (B), a refractive index $n_M$ of the polymerizable monomer component (A) with respect to the line D at 25°C is 1.490 to 1.550, and

when a refractive index at which a difference with $n_M$ is largest among the refractive indexes of at least one of the specific inorganic particulate matters (b1) occupying 10 mass% or more of the inorganic filler (B) is defined as $n_F$, an absolute value of a difference between $n_F$ and $n_M$ $|n_F - n_M|$ is 0.035 or less.

4. A method for producing a curable composition for three-dimensional photofabrication, comprising:

a mixing step of mixing 100 parts by mass of a polymerizable monomer component (A), 40 parts by mass to 400 parts by mass of an inorganic filler (B) containing a single type or a plurality of types of inorganic particulate matters, 0.01 parts by mass to 5 parts by mass of a photopolymerization initiator (C) having a function of initiating photopolymerization by irradiation with activating light including light having a specific wavelength $\lambda$ (nm) in an ultraviolet light range or a visible light range, and 0.01 parts by mass to 2.5 parts by mass of an activating light absorbent (D) having a function of absorbing the activating light and having no photopolymerization initiation ability,

wherein, in the mixing step, the polymerizable monomer component (A) and the inorganic filler (B) satisfy the following conditions 1 to 4:

condition 1: when a particle diameter of each particle constituting the inorganic filler (B) is defined as x (nm) and a circumference ratio is defined as $\pi$, a total number of particles having a particle diameter x (nm) within a range of $0.7 \lambda/\pi$ to $4 \lambda/\pi$ (nm) is 40% or more of the number of all particles constituting the inorganic filler (B) in a particle size distribution of the inorganic filler (B) measured by microscopy method using a scanning microscope;

condition 2: in the inorganic particulate matters constituting the inorganic filler (B), when an inorganic particulate matter consisting of an aggregate of a single type of inorganic particles having a refractive index with respect to a line D at 25°C in a range of 1.500 to 1.550 is defined as a specific inorganic particulate matter (b1) and an inorganic particulate matter consisting of an aggregate of a single type of inorganic particles having a refractive index out of the range is defined as a non-specific inorganic particulate matter (b2), the inorganic filler (B)

(1) contains a single type of the specific inorganic particulate matter (b1),
(2) contains a plurality of types of the specific inorganic particulate matters (b1), at least one of the plurality of types of specific inorganic particulate matters (b1) occupying 10 mass% or more of a total mass of the inorganic filler (B), or
(3) contains 90 mass% or more and less than 100 mass% of a single type or a plurality of types of the specific inorganic particulate matters (b1) and more than 0 mass% and less than 10 mass% of a single type or a plurality of types of the non-specific inorganic particulate matters (b2), at least one of the single type or the plurality of types of specific inorganic particulate matters (b1) occupying 10 mass% or more of a total mass of the inorganic filler (B);

condition 3: a refractive index $n_M$ of the polymerizable monomer component (A) with respect to the line D at 25°C is 1.490 to 1.550; and

condition 4: when a refractive index at which a difference with $n_M$ is largest among the refractive indexes of at least one of the specific inorganic particulate matters (b1) occupying 10 mass% or more of the inorganic filler (B) is defined as $n_F$, an absolute value of a difference between $n_F$ and $n_M$ $|n_F - n_M|$ is 0.035 or less.

5. The method for producing a curable composition for three-dimensional photofabrication according to claim 5, wherein the polymerizable monomer component (A) and the inorganic filler (B) satisfying the conditions 1 to 4 are used to separately prepare a base composition containing a composition which consists of the polymerizable monomer component (A) and the inorganic filler (B) and in which a composition ratio of the components is same as a composition ratio of the curable composition for three-dimensional photofabrication as a production target, and

the polymerizable monomer component (A) and the inorganic filler (B) found to have a value of a light scattering index Sc (%) of 10% or more are used in the mixing step, the light scattering index being determined by the following formula based on an intensity of transmitted light in a specific emission angle direction obtained by measurement using a variable angle spectrophotometer in which a sample having a thickness of 0.5 mm and containing the base composition is vertically irradiated with measurement light including light having the specific wavelength $\lambda$ (nm), including light having a wavelength in a range of $\lambda \pm 50$ (nm) as a main component, and having a maximum intensity in the range:

$$Sc = \{(I_{70} + I_{75} + I_{80})/(I_0 \times 3)\} \times 100$$

wherein $I_0$, $I_{70}$, $I_{75}$, and $I_{80}$ indicate intensities of the transmitted light in directions of emission angles of 0°, 70°, 75°, and 80°, respectively.

6. A method for producing a three-dimensional photofabricated product, which is a method for producing a three-dimensional photofabricated product by irradiating a predetermined position of a liquid photocurable composition held in a vat with activating light including light having a specific wavelength $\lambda$ (nm) in an ultraviolet light range or a visible light range and selectively curing the liquid photocurable composition present at the position, the method comprising:

a shaping step of digitizing and ordinating a height direction of a three-dimensional object based on three-dimensional shape data indicating a shape of the three-dimensional object, generating two-dimensional shape data indicating a cross-sectional shape of the three-dimensional object at each ordinated height, irradiating the liquid photocurable composition held in the vat with the activating light at a predetermined position determined in advance based on the two-dimensional shape data, thereby selectively primarily curing the liquid photocurable composition present at the position, forming a fabricated layer having the cross-sectional shape, and sequentially forming and stacking the fabricated layer having the cross-sectional shape at each height according to an order of the ordinating, to obtain a stack having a shape corresponding to the shape of the three-dimensional object;
a washing step of washing, with an organic solvent, the stack obtained in the shaping step; and
a secondary curing step of secondarily curing the stack washed in the washing step by performing additional activating light irradiation or a heating treatment, or both of the additional activating light irradiation and the heating treatment,
the curable composition for three-dimensional photofabrication according to claim 1 being used as the liquid photocurable composition.

7. A method for producing a dental restoration, comprising: producing the dental restoration by the method for producing a three-dimensional photofabricated product according to claim 6.

# FIG .1

# FIG .2

# FIG .3

500.000μm

# FIG .4

500.000μm

# FIG .5

RELATIVE LIGHT INTENSITY

300    350    400    450    500

WAVELENGTH OF TRANSMITTED LIGHT (nm)

## INTERNATIONAL SEARCH REPORT

| International application No. |
| --- |
| **PCT/JP2024/001193** |

### A. CLASSIFICATION OF SUBJECT MATTER

**B29C 64/314**(2017.01)i; **A61C 13/15**(2006.01)i; **A61K 6/802**(2020.01)i; **A61K 6/831**(2020.01)i; **B29C 64/165**(2017.01)i; **B29C 64/35**(2017.01)i; **B29C 64/379**(2017.01)i; **B33Y 10/00**(2015.01)i; **B33Y 40/20**(2020.01)i; **B33Y 70/10**(2020.01)i; **C08K 3/013**(2018.01)i; **C08L 101/12**(2006.01)i

FI:   B29C64/314; A61C13/15; A61K6/802; A61K6/831; B29C64/165; B29C64/35; B29C64/379; B33Y10/00; B33Y40/20; B33Y70/10; C08K3/013; C08L101/12

According to International Patent Classification (IPC) or to both national classification and IPC

### B. FIELDS SEARCHED

Minimum documentation searched (classification system followed by classification symbols)

B29C64/00-64/40; A61C5/20-5/35; A61C5/70-5/88; A61C8/00-13/38; A61K6/00-6/90; B33Y10/00-99/00; C08K3/00-13/08; C08L1/00-101/14

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | JP 2021-165375 A (CANON KABUSHIKI KAISHA) 14 October 2021 (2021-10-14) paragraphs [0036], [0057], [0063], [0067], [0076], [0079]-[0084] | 1-8 |
| A | WO 2016/076180 A1 (NIPPON ELECTRIC GLASS CO.) 19 May 2016 (2016-05-19) entire text, all drawings | 1-8 |
| A | WO 2016/084572 A1 (NIPPON ELECTRIC GLASS CO.) 02 June 2016 (2016-06-02) entire text, all drawings | 1-8 |
| A | JP 2020-044835 A (CANON KABUSHIKI KAISHA) 26 March 2020 (2020-03-26) entire text, all drawings | 1-8 |
| A | JP 2020-73333 A (NIPPON ELECTRIC GLASS CO.) 14 May 2020 (2020-05-14) entire text, all drawings | 1-8 |

☑ Further documents are listed in the continuation of Box C.    ☑ See patent family annex.

| | |
| --- | --- |
| *   Special categories of cited documents: | "T"   later document published after the international filing date or priority date and not in conflict with the application but cited to understand the principle or theory underlying the invention |
| "A"   document defining the general state of the art which is not considered to be of particular relevance | |
| "D"   document cited by the applicant in the international application | "X"   document of particular relevance; the claimed invention cannot be considered novel or cannot be considered to involve an inventive step when the document is taken alone |
| "E"   earlier application or patent but published on or after the international filing date | |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | "&"   document member of the same patent family |
| "P"   document published prior to the international filing date but later than the priority date claimed | |

| Date of the actual completion of the international search | Date of mailing of the international search report |
| --- | --- |
| **29 March 2024** | **09 April 2024** |

| Name and mailing address of the ISA/JP | Authorized officer |
| --- | --- |
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (July 2022)

**INTERNATIONAL SEARCH REPORT**

International application No.

**PCT/JP2024/001193**

| C. | DOCUMENTS CONSIDERED TO BE RELEVANT | |
|---|---|---|
| Category* | Citation of document, with indication, where appropriate, of the relevant passages | Relevant to claim No. |
| A | JP 2020-192810 A (NIPPON ELECTRIC GLASS CO.) 03 December 2020 (2020-12-03) entire text, all drawings | 1-8 |

Form PCT/ISA/210 (second sheet) (July 2022)

**INTERNATIONAL SEARCH REPORT**
Information on patent family members

International application No.

**PCT/JP2024/001193**

| Patent document cited in search report | | | Publication date (day/month/year) | Patent family member(s) | | | Publication date (day/month/year) |
|---|---|---|---|---|---|---|---|
| JP | 2021-165375 | A | 14 October 2021 | US | 2023/0040808 | A1 | |
| | | | | paragraphs [0042], [0063], [0069], [0073], [0093]-[0097], [0104]-[0106] | | | |
| | | | | WO | 2021/205954 | A1 | |
| WO | 2016/076180 | A1 | 19 May 2016 | JP | 2020-94223 | A | |
| | | | | JP | 2020-192810 | A | |
| | | | | US | 2017/0321056 | A1 | |
| | | | | CN | 107108768 | A | |
| WO | 2016/084572 | A1 | 02 June 2016 | (Family: none) | | | |
| JP | 2020-044835 | A | 26 March 2020 | US | 2020/0079896 | A1 | |
| JP | 2020-73333 | A | 14 May 2020 | (Family: none) | | | |
| JP | 2020-192810 | A | 03 December 2020 | US | 2017/0321056 | A1 | |
| | | | | WO | 2016/076180 | A1 | |
| | | | | CN | 107108768 | A | |
| | | | | JP | 2020-94223 | A | |

Form PCT/ISA/210 (patent family annex) (July 2022)

36

**REFERENCES CITED IN THE DESCRIPTION**

**Patent documents cited in the description**

- WO 2018074380 A
- JP 2020180171 A
- JP 2022041276 A
- JP 3917204 B